# EUROPEAN PATENT APPLICATION

(11) **EP 4 483 915 A1**
(43) Date of publication of application: **01.01.2025**
(21) Application number: 23181618.2
(22) Date of filing: 27.06.2023
(51) Int. Cl.: A61M 1/16, A61M 1/36, A61M 60/109, A61M 60/113, A61M 60/232, A61M 60/279, A61M 60/38, A61M 60/569

(54) **COMBINED BLOOD PUMP AND OXYGENATOR SYSTEM**

(71) Applicant: BIOxy Med LLC-FZ, Dubai (AE)
(72) Inventor: ABOULHOSN, Walid, 00000 Btekhnay (LB); KARAMEH, Fadi, 5447 AinZhalta (LB); RAFEH, Nidal Abi, Covington, Louisiana, 70433 (US); SALHA, Sary, 00000 Ras el Maten (LB); EL BASHA, Rawan, 5673 Dmit (LB); EL GHOSAYNI, Hadi, 5728 Baakleen (LB); SHKEIR, Faysal, 00000 Ras el Maten (LB); HADDAD, Jad, 00000 Ain dara (LB); HARB, Hasan, 00000 Gharifeh (LB); MAHMOUD, Yasser, 00000 Srayra (LB)
(74) Representative: Platzöder, Michael Christian

(57) **Abstract**

A blood pump-oxygenator system (1; ...; 10) comprises at least one blood pump (11), at least one oxygenator (20, 21), a blood flow inlet (80), and a blood flow outlet (90), which are connected so as to form a circuit to be operable as a cardiopulmonary bypass system adapted for extracorporeal processing of the patient's blood, wherein the first blood pump (11) is configured to convey blood through the circuit from the patient (P) into the blood flow inlet (80), through the oxygenator (20, 21) and out of the blood flow outlet (90) back into the patient (P). The system (1; ...; 10) further comprises a recirculation loop (100) arranged between the blood flow inlet (80) and the blood flow outlet (90), wherein the recirculation loop (100) accommodates the first blood pump (11) and the oxygenator (20, 21), wherein the first blood pump (11) is configured to circulate at least part of the blood in the circuit through the recirculation loop (100) such that the blood passes through the oxygenator (20, 21) multiple times such that the blood is repeatedly subject to the exchange of O₂ and CO₂ within the oxygenator (20, 21). At least a first valve (31) is provided positioned between the recirculation loop (100) and the blood flow outlet (90) and configured to control an amount of blood flowing through the recirculation loop (100).

## Description

### BACKGROUND OF THE INVENTION

### Technical Field

The present invention relates generally to the field of blood pumps and oxygenators used to increase perfusion and oxygen level in a patient. More particularly, the present invention can provide a pump-oxygenator system that is easy to set up and use, is compact in size and may allow for increased mobility of a patient.

### Background Art

Various types of blood pump-oxygenator systems are well known in the art. For example, during open-heart surgery, the patient is interconnected with an external pump-oxygenator system, commonly known as a heart-lung machine, which circulates blood and introduces oxygen into the blood system. Most types of blood pumps employ a roller or a centrifugal pump to convey blood in the patient's vascular system and/or through an extracorporeal system. Most types of oxygenators use a gas-permeable membrane, wherein blood flows along one side of the membrane and oxygen is supplied to the other side of the membrane. Given a sufficient pressure gradient between the oxygen supply and the blood, the oxygen will diffuse through the membrane and into the blood. In addition, carbon dioxide will tend to diffuse from the blood into the membrane. The membrane may be provided in the form of hollow fibers.

Such systems may be referred to as extracorporeal membrane oxygenation (ECMO), also known as extracorporeal life support (ECLS). This is an extracorporeal technique of providing prolonged cardiac and respiratory support to persons whose heart and lungs are unable to provide an adequate amount of gas exchange or perfusion to sustain life. The technology for ECMO is largely derived from cardiopulmonary bypass, which provides shorter-term support with arrested native circulation. Typical cardiopulmonary bypass systems are rather complex, and generally are not particularly well adapted for applications longer than six hours. Moreover, most standard systems exhibit poor hemodynamic characteristics. That is, such systems typically cause too much damage to the blood to be useful for extended periods.

Cardiopulmonary bypass systems are disclosed for example in US 3,890,969, US 4,466,804, and US 4,610,656. Such conventional systems commonly utilize several pumps, a venous reservoir, an arterial reservoir, and a separate bubble-trapping device. These conventional systems exhibit several disadvantages. The most apparent disadvantage is the overall complexity of such systems. For example, the numerous components require extensive tubing and interconnections.

The complexity of the conventional systems leads to higher costs of manufacture and operation. Also, complex systems may take longer to set up, and system set-up may require expert personnel and supervision. Even with such expert personnel present, a system's complexity increases the risk of error in setting up the system. Likewise, once in operation, a conventional system requires continuous monitoring and adjustment by expert personnel.

Conventional cardiopulmonary bypass systems are also not usable for long-term application because they significantly damage the blood after a fairly short use (e.g., 6-8 hours). For instance, conventional occlusive roller pumps mechanically destroy red blood cells. This "blood trauma" can occur in any cardiopulmonary bypass system. It is caused and/or aggravated by occlusive pumps, interconnections, and other system components likely to increase system pressure or turbulence. Similarly, using conventional oxygenators for beyond a few hours to a day results in diminished performance due to plasma leakage into the hollow fibers.

The above factors illustrate the need for and desirability of a simple cardiopulmonary bypass system with relatively few components. Devices similar to that disclosed in US 4,540,399 represent attempts to achieve simplicity. There it is disclosed an emergency bypass system with one non-occlusive pump, an oxygenator, and a separate bubble trap. It is described a centrifugal rotor-type pump connected proximal to (i.e., on the venous side of) the oxygenator. While this may represent an attempt to provide a simplified cardiopulmonary bypass system compared to other known systems, it still may be considered to need further improvement.

In other situations, a smaller, implantable oxygenator may be sufficient to adequately supplement the patient's cardiopulmonary function by marginally increasing the oxygen content of the patient's blood. For example, patients suffering from emphysema, pneumonia, congestive heart failure, or other chronic lung disease often have blood oxygen partial pressures of approximately 40 torr. A relatively small increase of 10% to 20% is generally sufficient to adequately maintain the patient. This is a particularly desirable alternative in that it can avoid the need to intubate the patient. In addition, temporary use of this type of oxygenator may be sufficient in many cases to tide the patient over an acute respiratory insult. Placing such patients on a conventional respirator is often the beginning of a progressive downhill spiral by damaging the patient's pulmonary tree, thereby causing greater dependence on the respirator.

A number of devices and processes have been invented in the past incorporating this basic technology, including the following. US 3,505,686 demonstrates the general concept of using gas permeable fibers to boost the oxygen level of blood. In the implantable embodiment of this disclosure, a tubular casing serves as a shunt either from the pulmonary artery to the left atrium of the heart, or more generally between an artery and a vein. A multitude of parallel-connected capillary tubes are used to oxygenate and/or purify the blood circulating through the casing. US 4,583,969 shows a transvenous oxygenator made of a plurality of small diameter gas permeable tubes. However, in this specific device disclosed two incisions may be required. The insertion process is also rather complex. US 4,631,053 discloses a transvenous oxygenator having a single membrane through which oxygen diffuses. The membrane is disposed within a sheath and a flexible wire supports both.

WO 2021/170712 A1 discloses a combined blood pump and oxygenator system and related methods. A blood pump-oxygenator system is provided that comprises at least one blood pump, an oxygenator, an inflow cannula, and an outflow cannula, which are connected so as to form a closed series circuit to be operable as a cardiopulmonary bypass system adapted for extracorporeal processing of the patient's blood. The blood pump is configured to convey blood through the circuit from the patient into the inflow cannula, through the oxygenator and out of the outflow cannula back into the patient. A manifold is connected between the inflow cannula and the outflow cannula so that the blood passes through the manifold, wherein the manifold accommodates the blood pump and the oxygenator and is designed so as to form a recirculation loop configured to recirculate at least part of the blood in the circuit such that the blood passes over the oxygenator multiple times. The system further comprises an extra blood pump positioned at the outflow cannula and configured to deliver a set volume to the patient, wherein the extra pump is configured to be controlled independently from the blood pump that circulates the blood in the manifold including the oxygenator.

Recently, a number of centers are implementing ambulatory ECMO support using available pumps and oxygenators, allowing patients to walk, eat and exercise. This is an improvement because it allows patients to move, and participate in physical rehabilitation, preventing muscles from atrophy and ultimately leading to a better clinical course to recover or have a better status at the time of heart/lung transplantation.

Currently available devices may be very bulky and multiple exchanges of oxygenators are required for long-term support, which usually cannot be done without the interruption of the whole system. Moreover, the rigid nature of the currently available oxygenators usually defines the circulation track of the blood, which restricts the circulation of blood to be divided all over the area of oxygenator. This causes fast decay of the same hollow fibers where the blood is always circulating, and decreases the lifetime of the system as a whole.

A need exists for a circulatory support system capable of supporting a patient in circulatory dysfunction for some length of time. Such a circulatory support system could be quite useful in numerous situations, such as (1) where the patient is in a state of cardiogenic shock; (2) where the patient is in a state of septic shock, (3) for post cardiotomy weaning from the bypass, (4) for assisting the circulatory system to avoid an impending myocardial infarction; and (5) bridgeto-transplant patients. It may further be desirable to provide a circulatory support system that can be used in an ambulatory environment and provides mobility for the patient.

### SUMMARY OF THE INVENTION

It is an object of the present invention to provide an improved blood pump and oxygenator system. Specifically, the present invention is directed at addressing at least one of the above-mentioned needs and eliminating, or at least reducing, at least one of the shortcomings of the prior art systems as described above.

A solution to this problem is provided by the teaching of the independent claims. Various preferred embodiments of the present invention are provided by the teachings of the dependent claims.

According to one aspect, a blood pump-oxygenator system comprises a first blood pump, at least one oxygenator, a blood flow inlet, and a blood flow outlet, which are connected so as to form a circuit to be operable as a cardiopulmonary bypass system adapted for extracorporeal processing of the patient's blood, wherein the first blood pump is configured to convey blood through the circuit from the patient into the blood flow inlet, through the oxygenator and out of the blood flow outlet back into the patient. The system further comprises a recirculation loop arranged between the blood flow inlet and the blood flow outlet, wherein the recirculation loop accommodates the first blood pump and the oxygenator(s), wherein the first blood pump is configured to circulate at least part of the blood in the circuit through the recirculation loop such that the blood passes through the oxygenator(s) multiple times. At least a first valve is positioned between the recirculation loop and the blood flow outlet and configured to control an amount of blood flowing through the recirculation loop.

The system of the present invention allows for recirculation of the blood (or at least part of the blood in the system) such that the blood is repeatedly subject to the exchange of O₂ and CO₂ within the oxygenator(s). The valve is controlled to set an amount of blood that is recirculated in the recirculation loop, which in turn at the same time determines an amount of blood that is delivered back to the patient. By controlling the valve and thus the amount of recirculated blood, oxygenation can be significantly improved compared to conventional systems in which the blood passes through the oxygenator(s) only once. The blood passes the blood over the oxygenator multiple times, thereby providing the same level of oxygenation and allowing the size of the oxygenator to be greatly reduced in size relative to "single pass" oxygenators. The "multiple pass" feature of the present invention is also advantageous in that it serves to provide a heat exchanger functionality as well as air entrapment. It will be appreciated that a control unit may be provided to control the first blood pump and the first valve as well as further controllable components of the system mentioned below.

By varying the amount of blood in the recirculation loop, the blood will tend to pass through the oxygenator(s) along different paths within the oxygenator. This further improves oxygenation of the blood by utilizing the full capacity of the oxygenator(s). Apart from that, the lifetime of the oxygenator(s) can be prolonged because different areas of the oxygenator(s) are used in the oxygenation process, which achieves a regular wear of the oxygenator. This is advantageous compared to a system in which basically always the same areas of the oxygenator(s) are used while other areas may not be used (or used very little).

The system of the present invention can provide partial veno-arterial cardiopulmonary support over a relatively long period (i.e., one to ten days) because of its very low overall blood trauma and improved ease of use characteristics. The system is capable not only of providing cardiopulmonary support but also of providing hemodynamic support over a potentially long period. The system of the present invention is particularly adapted for partial hemodynamic support. Notwithstanding this, it is to be readily appreciated that the system is also suitable for achieving a number of additional objectives in a variety of situations involving circulatory dysfunction. The system of the present invention can assist circulation during relatively mild circulatory dysfunction, and also can provide acute assistance during severe refractory cardiac failure caused by myocardial infarction, cardiomyopathy, or post-cardiac surgery. The system of the present invention may also be useful in providing perioperative support and stabilization of high-risk patients, such as coronary bypass surgery patients.

The system of the present invention may also be useful in causing less damage to the blood than conventional systems by reducing the surface area the blood interacts with, making the system of the present invention more suitable for long-term applications. Trauma to the patient's blood can be reduced principally by the simplicity of a blood circuit, especially a low number of circuit elements, a non-occlusive pump and a design of the oxygenator(s). In addition, overall trauma to the patient can be reduced. This can be accomplished by reducing the extracorporeal processing of the blood as compared to conventional bypass systems and procedures. The absence of separate fluid reservoirs can reduce the fluid volume of the system. Because less of the patient's blood is withdrawn from the body at one time, the overall trauma to the patient can be reduced.

The low trauma of the system contributes desirably to overall hemodynamic characteristics. Many of the features of the present invention that improve the overall hemodynamics of the system may also help to simplify the system, and make it more reliable and compact. To further reduce the risk of system malfunction, the system may be equipped with various fail-safe features. Such equipment may be used to monitor and control various pressures and flow rates to ensure proper operation. Although the system of the present invention may require periodic monitoring and adjustment by expert personnel during operation, it can be operated relatively automatically as will be described in more detail below. That is, compared to conventional systems, which tend to be more complex, the system of the present invention requires much less monitoring and adjustment during operation.

The term "oxygenator", as used herein, particularly refers to a medical device that is configured to oxygenate blood and remove carbon dioxide from the blood. It is used to maintain gas exchange, e.g., in cardiac surgery as part of a heart-lung machine or in critical care medicine to treat acute respiratory failure. The oxygenator briefly replaces the function of the lungs. The oxygenator may be specifically a membrane oxygenator. Such oxygenator may have hollow fibers, such as hair-like fibers. The fibers may be arranged in "sets" or "bundles", wherein fibers of one set may have a common inlet and outlet respectively. The oxygenator may comprise a plurality of hollow oxygenation fibers that allow for blood to pass between the oxygenator fibers to intake oxygen and release carbon dioxide, while oxygen is passed through an inside lumen of the oxygenator fibers from the inflow lumen at one end of the oxygenator fibers and returned from the other end of oxygenator fibers to the outflow lumen. Oxygen may be delivered to the oxygenator via an inflow tube connected to the inflow, and an outflow tube connected to the outflow may be provided for removal of carbon dioxide from the oxygenator.

The term "blood pump" (or simply "pump"), as used herein, particularly refers to a medical device for pumping blood, specifically through an extracorporeal system. The blood pump may be a rotary pump or a displacement pump, such as a rotary pump of the centrifugal type or the axial type, wherein the blood pump may be configured to be directly driven by an electric motor or to be driven by a flexible drive cable that links the blood pump to the electric motor either magnetically or directly. A rotor may be situated inside a pump housing, wherein a drive cable may be inside a sheath coupled to the rotor in such a manner that rotation of the drive cable by an electric motor causes rotation of the rotor and pumping of blood from the distal end to the proximal end of housing so as to transport the blood from the blood flow inlet toward the blood flow outlet.

If applicable, the terms "first", "second", "third" and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Where the term "comprising" or "including" is used in the present description and claims, it does not exclude other elements or steps. Where an indefinite or definite article is used when referring to a singular noun e.g., "a" or "an", "the", this includes a plural of that noun unless something else is specifically stated.

Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

In the following, preferred embodiments of the method are described, which can be arbitrarily combined with each other or with other aspects of the present invention, unless such combination is explicitly excluded or technically impossible.

In some embodiments, the system further comprises a second blood pump positioned between the recirculation loop and the blood flow outlet and configured to deliver a set volume to the patient, wherein the second blood pump is preferably configured to be controlled independently from the first blood pump that circulates the blood in the recirculation loop. Providing two blood pumps rather than only one may reduce hemolysis because the pressure in the circuit is controlled by two pumps. Therefore, the flow in each of the pumps can be lower which causes less blood damage.

In some embodiments, the first blood pump is configured to be set to deliver a flow rate in excess of what is desired to deliver to the patient, wherein the excess amount of blood (specifically all excess amount of blood) is recirculated through the recirculation loop and the oxygenator. In case a second blood pump is provided, the second blood pump may be configured to deliver the desired amount of blood to the patient. This configuration may improve the recirculation of at least part of the blood in the recirculation loop as there is an excess amount of blood provided at all times. This excess amount of blood may be recirculated and may thus pass the oxygenator multiple times. It will be appreciated that there may be a continuous addition and withdrawal of blood to and from the recirculation loop to provide a continuous circulation in the overall system.

In some embodiments, the system comprises at least two oxygenators (i.e., at least one additional oxygenator), wherein the recirculation loop comprises a bifurcation that diverts the blood flow in the recirculation loop into at least two branches arranged in parallel with each other and a junction where the at least two branches are brought together, wherein one of the at least two oxygenators is arranged in each of the at least two branches. More specifically, a "main section" of the recirculation loop is split up at the bifurcation into parallel branches, wherein the main section may be considered a section of the recirculation loop that directly connects the blood flow inlet with the blood flow outlet bypassing the oxygenator(s), and the branches are brought together at the junction leading back into the main section of the recirculation loop. Using two (or more) oxygenators in parallel allows to reduce the size of each of the oxygenators. The dwell time of the blood in the oxygenators can be increased, particularly doubled, by providing at least two oxygenators.

In some embodiments, at least one of the bifurcation and the junction is configured as a multi-way control valve that is operable so as to select at least one of the at least two branches for the blood to flow through. The provision of multi-way control valves allows to control the proportion of blood that flows through each branch, and thus through each of the oxygenators. In particular, the valves may be controlled such that blood flows only through one of the branches at the same time. If multi-way vales are provided at each of the bifurcation and the junction, i.e., at the beginnings and ends of the parallel branches of the recirculation loop, the valves may be synchronized to establish a flow path through only a single branch at the same time. Thus, the dwell time of the blood in one oxygenator can be increased, while the other oxygenator is emptied. Furthermore, an oxygenator can be replaced easily by blocking the respective branch of the recirculation loop, while the other branch of the recirculation loop, and thus the other oxygenator, can continue to operate. The possibility of swapping oxygenators without the need to stop the system increases the usefulness of the system for long-term applications. Oxygenators in general tend to degrade in function very rapidly over time. Therefore, the possibility to change oxygenators without interrupting patient support may increase the overall lifetime of the system.

A multi-way control valve may have multiple ports (openings), wherein one of the ports may act as an inlet or outlet whereas the remaining ports act respectively as outlets and inlets respectively. For instance, a three-way control valve has three ports, through whose openings volume flows enter or leave. They may be used as mixing valves or as diverting valves (also referred to as switching valves). The flow rate may be regulated by an electric, pneumatic or electro-pneumatic actuator. A three-way mixing valve has two inlets and one outlet, wherein two flows, particularly the flows from the branches at the junction, flow into the valve and leave it mixed via the third way. In a diverting valve (one inlet and two outlets), the incoming volume flow is distributed to two outlets, leading to the branches at the bifurcation.

In some embodiments, the first blood pump is arranged in the recirculation loop upstream of the bifurcation (i.e., in a flow direction of the blood "before" the bifurcation). In contrast to that, in some other embodiments the system comprises at least two of the first blood pump, wherein one of the at least two first blood pumps is arranged downstream of the bifurcation (i.e., in a flow direction of the blood "after" the bifurcation) in each of the at least two branches, preferably in each of the at least two branches between the bifurcation and the respective one of the at least two oxygenators.

In some embodiments the junction is configured as a reservoir, wherein the reservoir comprises at least two inlet ports, an outlet port and at least one vent. Each of the branches ends in the reservoir at a respective inlet port such that all blood from the branches and thus the oxygenators is fed to the reservoir. The collected blood exits the reservoir at the outlet port and is further recirculated, wherein the outlet port is extended towards an inside of the reservoir. The at least one vent is configured for exhausting gas bubbles in the blood in the reservoir. Thus, the reservoir may also be referred to as gas bubble removing device. It is advantageous to remove bubbles at this point in the system by means of the reservoir because it can be avoided that gas bubbles are further circulated even if gas bubbles may be removed by a bubble trap at the blood flow outlet as described below. It will be appreciated that in more general terms the reservoir may also be provided in embodiments having only one oxygenator, wherein the reservoir has only one inlet port in this case, wherein the reservoir is arranged in the recirculation loop after the oxygenator.

Particularly, a basic principle for removing gas bubbles may be that the reservoir is in a position during operation where the vent(s) is/are in an upper portion thereof as the gas bubbles will rise upwards. The inlet port(s) is in an upper portion, too, while the outlet port is designed in such a way that it takes blood from a location that is below the vent, e.g., by means of a tube or the like that extends to the insidd of the reservoir, e.g., towards the center or further to a lower portion of the inside of the device. Preferred embodiments will be described in more detail below with respect to the drawings.

In some embodiments, the system further comprises a first oxygen sensor and a second oxygen sensor both arranged in the recirculation loop and configured to measure an oxygen level in the blood, wherein the first oxygen sensor is positioned upstream of the oxygenator(s) and the second oxygen sensor is positioned downstream of the oxygenator(s) so as to obtain an oxygenation rate of the oxygenator(s) by a comparison of an oxygen level measured by the first oxygen sensor with an oxygen level measured by the second oxygen sensor. This allows to monitor the function of the oxygenator(s). It will be appreciated that in case of two or more oxygenators, a second oxygen sensor may be provided downstream of each oxygenator to be able to monitor the oxygenation level of the blood after it has passed the respective oxygenator. A single oxygen sensor upstream of the oxygenators may be sufficient.

In some embodiments, the first blood pump is configured to be controlled such that a pump rate of the first blood pump is set depending on the obtained oxygenation rate, wherein preferably a pump rate of the first blood pump is increased when the oxygenation rate decreases. The obtained oxygenation rate can be useful to control the system. In particular, if it is detected that an oxygenator provides less or decreasing oxygenation, the pump rate can be increased to increase the amount of blood passing through the oxygenator and to maintain the level of oxygen.

In some embodiments, the first valve is configured to be controlled such that an amount of blood flowing through the recirculation loop is set depending on the obtained oxygenation rate, wherein preferably a flow rate of the first valve is decreased when the oxygenation rate decreases. As described above, the obtained oxygenation rate can be useful to control the system. In particular, if it is detected that an oxygenator provides less or decreasing oxygenation, the valve can be controlled accordingly to increase the amount of blood passing through the recirculation loop and, thus, the oxygenator to achieve a desired level of oxygen.

In some embodiments, the system further comprises a second valve positioned between the blood flow inlet and the recirculation loop and configured to control an amount of blood flowing from the patient into the circuit. In some embodiments, at least one of the first valve and the second valve may be configured to be synchronized with the heart rhythm, wherein preferably the valve is fully opened during systole and fully closed during diastole.

In some embodiments, the system further comprises a first pressure sensor positioned at the blood flow inlet and configured to measure an input pressure of the circuit. A pressure sensor at the blood flow inlet is particularly advantageous to monitor the input pressure of the system. In particular, it should be avoided to have negative pressures in the system and specifically in the pump, which could cause gas bubbles in the blood.

In some embodiments, the first blood pump is configured to be controlled such that a pump rate of the first blood pump is set depending on the measured input pressure, wherein preferably a pump rate of the first blood pump is increased when the input pressure drops to a predefined threshold. As mentioned above, this is particularly useful to avoid negative pressures. The measured input pressure can provide an input parameter for the first blood pump which draws blood into the system.

In some embodiments, the system further comprises a second pressure sensor positioned at the blood flow outlet and configured to measure an output pressure of the circuit. It is also of importance to monitor a pressure for the blood that is delivered back to the patient in order to avoid harm to the patient, e.g., by a too high output pressure.

In some embodiments, the system further comprises a third pressure sensor and a fourth pressure sensor both arranged in the recirculation loop, wherein the third pressure sensor is positioned upstream of the oxygenator(s) and the fourth pressure sensor is positioned downstream of the oxygenator(s) so as to detect a pressure difference, specifically a pressure drop, caused by the oxygenator(s) by a comparison of a pressure measured by the third pressure sensor with a pressure measured by the fourth pressure sensor. Monitoring the pressure before and after the oxygenator(s) allows to monitor the function of the oxygenator(s). In particular, if an oxygenator is blocked, a pressure drop may occur. This malfunction can then be detected easily and quickly, and the respective oxygenator can be replaced.

In some embodiments, the system further comprises at least one flow meter to measure an amount of blood flow, the at least one flow meter comprising at least one of a first flow meter and a second flow meter, the first flow meter arranged in the recirculation loop, preferably between the first blood pump and the oxygenator(s), and configured to measure a flow rate of blood in the recirculation loop, and the second flow meter arranged between the recirculation loop and the blood flow outlet and configured to measure a flow rate of blood that is delivered back to the patient. Various flow meters may be provided throughout the system, preferably at locations where the pressure sensors are arranged. Measurement data of a pressure sensor together with measurement data of a flow meter provide detailed information about the condition and function of the system.

In view of the aforementioned, the system may be considered to have a pressure feedback system particularly to control pump suction force of the blood pump(s). The pump-oxygenator system of the present invention may be provided with a pressure feedback system to control the pump suction force also to eliminate the need for separate reservoirs. In this regard, the system may be a relatively static-volume system as compared to conventional systems, which normally include one or more separate reservoirs or the like. The pump-oxygenator system may be equipped with an electric heater and thermal insulation so as to eliminate the need for a heat exchanger to maintain fluid temperature. The reduced size of the system and the reduced dwell time of the blood outside the patient's body may also eliminate the need for a heat exchange system.

In some embodiments, the system further comprises a bubble trap at the blood flow outlet, wherein the bubble trap is configured to receive any blood before it is delivered back to the patient through the blood flow outlet and to retain any gas bubbles before the blood leaves the bubble trap.

In some embodiments, the need for a separate bubble trap may be eliminated in that negative pressure is used to circulate gas in the oxygenator. The pump-oxygenator may be positioned such that the fluid may flow through the oxygenator to the inlet of the pump by gravity, or by gravity in combination with the patient's venous pressure. This orientation further enables a smooth and low-pressure fluid flow through the system, further reducing blood trauma.

According to another aspect, a wearable soft shell extracorporeal pump-oxygenator system is provided, which may be provided with the features described herein for the blood pump-oxygenator system. The wearable soft shell extracorporeal pump-oxygenator system can function either as a respiratory support device or cardiopulmonary support device with flexibility of application in the broad spectrum of heart/lung disease, particularly as a temporary short or long-term bridge to transplantation or more aggressive medical therapies, which may extend patient survival.

Rather than rigid oxygenators, the oxygenators of the wearable system may use a flexible shell instead of a hard shell (meaning the oxygenator shell is compliant). Selector valves may be provided to select one flexible oxygenator or the other at a time. So, one flexible oxygenator can be filled from the pump while its output is closed. Therefore, one oxygenator may be filling while the other is emptying into the patient. In other words, one soft shell oxygenator may be inflating with blood while the other is emptying the oxygenated blood into the patient.

In some embodiments, a wearable softshell pump-oxygenator system is provided in the form of a wearable piece, particularly for the upper body (torso) of a patient, such as a vest, jacket, shirt, belt or the like. It will be appreciated that other types of pieces that can be worn be a patient in a suitable manner can be provided. The wearable piece, such as the vest, may comprise an outer shell and a blood oxygenator placed inside. The blood oxygenator may comprise or be composed of hollow fibers and may extend between a first and a second pump, wherein an inlet of the first pump may be connected to a patient's vessel, e.g., the patient's vein, and an outlet of the second pump may be connected to a vessel, e.g., the patient's artery. A circuit is thereby provided that provides continuous blood communication between components. The wearable system allows for mobility of a patient because of the portability of the ECMO device. For this purpose, the oxygenator(s) may have a flexible design, e.g., as a vest or the like as mentioned above.

The wearable system, like the system described above, can provide partial veno-arterial cardiopulmonary support over a relatively long period of time (i.e., one to ten days, up to 30 days) because of its very low overall blood trauma and improved ease of use characteristics. The system may be capable not only of providing (veno-venous or veno-arterial) cardiopulmonary support but also of providing hemodynamic support over a potentially long period of time (e.g., 1 to 30 days). Notwithstanding this, it is to be readily appreciated that the system is also suitable for achieving a number of additional objectives in a variety of situations involving circulatory dysfunction. The system can assist circulation during relatively mild circulatory dysfunction, and also can provide acute assistance during severe refractory cardiac failure caused by myocardial infarction, cardiomyopathy, or post-cardiac surgery. The system may also be useful in providing perioperative support and stabilization of high-risk patients, such as coronary bypass surgery patients.

In any of the above-described systems, pump operation could be cyclically operated in timed relation to patient's heartbeat or one of the physiological parameters, and a control circuit varies the velocity of the pump function in response to changes in the said physiological parameter. The device itself, controller, drive line and power supply can be worn by the patient (i.e., are portable) so that the patient can be ambulatory. This may significantly improve mobility of the patient compared to bulky stationary devices. As described above, blood enters through an inflow cannula, gets oxygenated through the oxygenator, such as the hollow fiber area that may be spread in almost complete area of the left side of the vest, which may have a capacity of around 0.4 to 0.6 liters, e.g., 0.5 liters. Around 2 L/min to 4 L/min, such as about 3 L/min of oxygenated blood may be delivered to the patient through a pump placed in the outflow cannula or at the bottom of the left side of the vest. The pump that delivers blood to the oxygenator may be configured to deliver around 8 L/min to 11 L/min, such as 9 L/min, which may be 3 L coming from patient and 6 L from the device inside the vest in a recirculation mode.

With regards to the access to the patient's blood circulation, any of the above described systems can be used in standard ECMO procedures, i.e., no specific instruments, access, introducers, etc. are required. For instance, suitable cannulas can be placed percutaneously in desired blood vessels, such as veins and/or arteries, by the Seldinger technique, or via surgical cutdown.

It will be appreciated that explanations, embodiments, and advantages described above in connection with one aspect may similarly apply to any other aspect of the invention.

### BRIEF DESCRIPTION OF THE DRAWINGS

Illustrative embodiments are described below with reference to the drawings. In the interest of clarity, not all features of an actual implementation may be described in this specification. It will of course be appreciated that in the development of any such actual embodiment, numerous implementation-specific decisions might be made to achieve the developers' specific goals, such as compliance with system-related and business-related constraints, which will vary from one implementation to another. In the drawings:
Fig. 1 shows an embodiment of a pump-oxygenator system having one oxygenator and one pump;
Fig. 2 shows an embodiment of a pump-oxygenator system having one oxygenator and two pumps;
Fig. 3 shows another embodiment of a pump-oxygenator system having one oxygenator and two pumps;
Fig. 4 shows an embodiment of a pump-oxygenator system having two oxygenators and one pump;
Fig. 5 shows an embodiment of a pump-oxygenator system having two oxygenators and two pumps;
Fig. 6 shows another embodiment of a pump-oxygenator system having two oxygenators and two pumps;
Fig. 7 shows an embodiment of a pump-oxygenator system having two flexible oxygenators and one pump;
Fig. 8 shows an embodiment of a pump-oxygenator system having two flexible oxygenators and two pumps;
Fig. 9 shows another embodiment of a pump-oxygenator system having two flexible oxygenators and two pumps;
Fig. 10 shows another embodiment of a pump-oxygenator system having two oxygenators and two pumps, each associated with one of the oxygenators;
Fig. 11 shows an embodiment of a pump-oxygenator system having two oxygenators and three pumps, two of which are each associated with one of the oxygenators;
Fig. 12 shows an embodiment of a pump-oxygenator system having two oxygenators and one pump having another valve configuration at the junction of the branches of the recirculation loop;
Fig. 13 shows an embodiment of a pump-oxygenator system having two oxygenators and one pump as well as a reservoir at the junction of the branches of the recirculation loop;
Fig. 14 shows an embodiment of a reservoir having a ball shape and multiple vents;
Fig. 15 shows a part of a wearable oxygenator system having multiple oxygenators;
Fig. 16 shows an embodiment of a wearable soft-shell pump-oxygenator system in the form of a vest comprising a blood oxygenator placed inside the vest;
Fig. 17 shows a zoom view of the first pump circuit of the embodiment of Fig. 16;
Fig. 18 shows a detailed view of the left side of the vest with inflow and outflow of blood correspondingly;
Fig. 19 shows another embodiment of a pump circuit forming a recirculation loop;
Fig. 20 shows a detailed view of two sets of hollow fibers;
Fig. 21 shows a detailed view of one set of hollow fibers;
Fig. 22 shows a side path of CO₂ out;
Fig. 23 shows another structure where CO₂ side path is bigger than in Fig. 17;
Fig. 24 shows another embodiment of a wearable system where there is a partition between the two sets of hollow fibers, blood will circulate from one side to the other through a small opening;
Fig. 25 shows another embodiment of a wearable system where there are provided multiple sets of hollow fibers, preferably four, two in each side of the vest;
Fig. 26 shows another embodiment of a wearable system in which the vest has hollow fibers in the back side of it as a backup in case the fibers in the front of the vest need to be changed;
Fig. 27 shows an embodiment similar to that of Fig. 26 but the fibers are in right and left sides of the back side of the vest with circulation of blood between the left and right;
Fig. 28 shows an embodiment of a wearable system where the back side of the vest is one piece that has hollow fibers all over;
Fig. 29 shows an embodiment of a wearable system, in which a hard cover is added to the device (any form mentioned in this disclosure) over the front vest in order to protect the components inside the vest; and
Fig. 30 shows an embodiment of a wearable system, in which a hard cover is added also over the back side of the vest in case it contains components.

### DESCRIPTION OF EMBODIMENTS

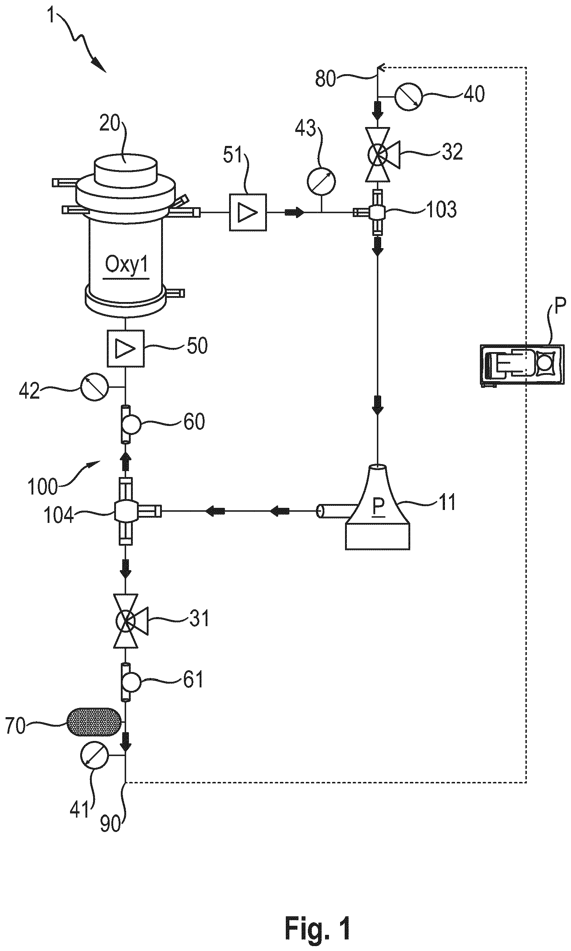

With reference to Fig. 1, an extracorporeal blood pump-oxygenator system 1 (hereinafter also simply referred to as "system") is provided comprising a blood pump 11 and an oxygenator 20. Blood is drawn from a patient P and enters the system 1 at a blood flow inlet 80 and is delivered back to the patient P at a blood flow outlet 90. The blood flow inlet 80 and blood flow outlet 90 may be provided by means of an inflow cannula 80 and an outflow cannula 90, respectively. Cannulas 80, 90 may be any suitable cannula of known construction for use in transporting blood. Cannulas 80, 90 may be made from any suitable biocompatible material, including but not limited to urethane or silicone or similar biocompatible material. Cannulas 80, 90 may be reinforced with metallic wire or similar material to help the cannula resist kinking when bent in a sharp radius. All these details are well known in the cannula manufacturing industry and are included here for clarity.

The blood pump 11 may be provided (by way of example only) as a rotary or a displacement pump and, more specifically, a rotary pump of the centrifugal type. Centrifugal pumps are a known art in the blood pumping area and are preferred due to the low cost of manufacturing and the low trauma to the blood. Blood pump 11 may be directly driven by an electric motor or driven by a flexible cable that links the pump to the electric motor (as described, for example, in US 4,944,722). The coupling between the pump and the driving mechanics may be magnetically or directly coupled. The pump 11 is shown of the centrifugal type with a rotor situated inside a pump housing. The pump may be driven by a drive cable inside a sheath coupled to the rotor (not shown). The rotation of the drive cable, by an electric motor (not shown), causes the rotation of the rotor and the pumping of fluid (blood) through the pump housing. In this fashion, fluid is transported from inflow cannula 80 toward outflow cannula 90. Specifically, the pump may create a suction force that draws blood from the patient P into the system 1 through the inflow cannula 80 and that conveys the blood from the inflow cannula 80 to the outflow cannula 90.

The blood pump 11 not only conveys the blood from the inflow cannula 80 to the outflow cannula 90 but also circulates at least part of the blood through a recirculation loop 100 of the system 1. Respective connections 103, 104 are provided to connect the recirculation loop 100 between the blood flow inlet 80 and the blood flow outlet 90. It is to be noted that both, the blood pump 11 and the oxygenator 20 are located in the recirculation loop 100. In this way, blood can be circulated through the oxygenator 20 multiple times to improve the overall oxygenation of the blood.

In essence, the system is designed to drain venous blood and infuse oxygenated blood during heart diastole and to re-circulate blood past the oxygenator during heart systole. This will advantageously result in the heart pumping against lower systemic pressure, which translates into a lower workload on the myocardium.

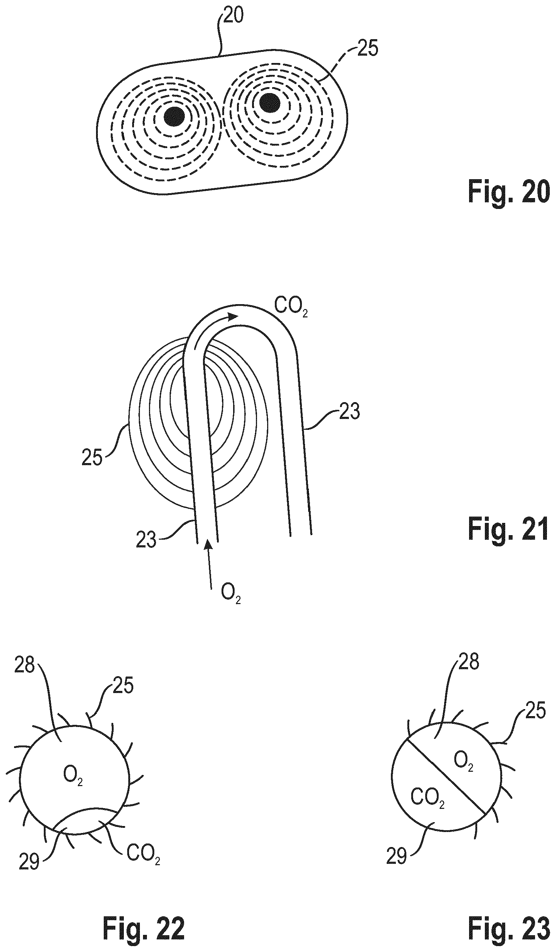

A typical oxygenator would comprise at least two lumens, an inflow lumen 28 for oxygen delivery to oxygenator fibers 25 (cf. Figs. 20 to 23) and an outflow lumen 29 for the removal of carbon dioxide (CO₂ gas) from the oxygenator fibers 25. Oxygenator 20 is provided with oxygenation fibers 25 that allow for blood to pass between oxygenator fibers 25 to intake oxygen and release CO₂ gas. In return, oxygen is passed through the inside lumen of oxygenator fibers 25 from inflow lumen 28 at one end of oxygenator fibers 25 and returned from the other end of oxygenator fibers 25 to lumen 29 of sheath 23 for release into the atmosphere. The pump 11 forces the blood to flow through oxygenator fibers 25 to maximize blood oxygen and CO₂ exchange. It may be desired at times to use a vacuum source to drive the flow of oxygen in the system rather than positive pressure. Vacuum driving will favor blood entry into the micropores of oxygenation fibers 25 rather than air leaving into the blood.

The pump 11 may be set to deliver a flow rate in excess of what is desired to deliver to the patient P, wherein the excess flow is re-circulated through the oxygenator 20 such that the blood is repeatedly subject to the exchange of O₂ and CO₂ within the oxygenator 20. The oxygenator 20 may be of substantially reduced size based on this "multiple pass" feature, wherein blood is passed multiple times over the oxygenator 20 before being circulated to the patient P. This "multiple pass" aspect may simplify the system and reduce its size and the blood volume needed to prime the circuit. Another advantage is the capability to place the circuit on the patient (see also description of flexible / wearable devices below) and therefore minimize the entire circuit complexity and size.

In order to be able to control the flow of blood through the system 1, suitable valves 31, 32 are provided that may be electrically or mechanically controlled depending on a desired or needed flow of blood. The system 1 comprises a first valve 31 between the recirculation loop 100 and the blood flow outlet 90 and a second valve 32 between the blood flow inlet 80 and the recirculation loop 100. The first valve 31 controls the portion of blood that returns to the pump inlet (i.e., the portion of the blood that circulates through the recirculation loop 100) and the portion of blood that flows to the patient P via the blood flow outlet 90. The second valve 32 controls the amount of blood that is drawn from the patient P into the system via the blood flow inlet 80.

Blood has the preference to re-circulate in the recirculation loop 100 to the pump inlet since it is the least resistive path for the blood flow. As such, the more the valve 31 is opened, the more flow will be diverted to the patient P. Stated another way, the valve 31 determines the ratio of blood returning to the pump 11 through the recirculation loop 100, versus the new blood coming from inflow cannula 80. The rotational rate of the pump 11 determines the total volume of blood flowing through pump 11. A high flow rate is typically desired since the higher the flow rate of pump 11 relative to outflow cannula 90, the more oxygenation will take place. Similarly, the higher the re-circulating blood portion through the recirculation loop 100, the smaller the oxygenator 20 may be since the same blood will pass multiple times over oxygenator 20 and get a higher level of oxygenation.

Apart from the pump 11, the oxygenator 20 and the valves 31, 32, the system 1 of Fig. 1 further comprises a variety of sensors. Specifically, pressure sensors 40, 41, 42, 43, flow meters 60, 61 and oxygen sensors 50, 51 are provided to obtain operational data of the system. Thereby, feedback loops can be designed to control operation of the pump 11, the valves 31, 32, and the oxygenator 20, more specifically to provide a system 1 that is able to be operated substantially fully automatically.

A first pressure sensor 40 is located at the blood flow inlet 80 to detect an input pressure of the system 1. For proper function, it is important that no high negative pressure occurs in the system 1, and specifically in the pump 11, as the blood will then tend to evaporate, which may cause gas bubbles. The pump 11 may be controlled depending on the measured input pressure. A second pressure sensor 41 is located at the blood flow outlet 90 to measure an output pressure of the blood that is delivered back to the patient P. Specifically, the pressure of the blood that is delivered into the patient's veins must not be too high. A flow meter 61 may also be provided near this pressure sensor 41 to obtain data of the amount of blood that leaves the system 1 (e.g., provided in liters per minute (L/min)).

Further pressure sensors 42, 43 are provided in the recirculation loop 100, one upstream of the oxygenator and one downstream. By comparing these measured pressures, more specifically the pressure of the blood before entering the oxygenator 20 and the pressure of the blood after it has passed through the oxygenator 20, an indication of the function of the oxygenator 20 may be provided. For instance, a pressure drop may indicate that the oxygenator 20 is blocked. Also, a flow meter 60 may be provided at the inlet side of the oxygenator 20 to measure an amount of blood that is circulated through the recirculation loop 100 (e.g., provided in L/min).

Oxygen sensors 50, 51 are provided in the recirculation loop 100, one upstream of the oxygenator and one downstream. By comparing the measured oxygen levels, more specifically the oxygen level of the blood before entering the oxygenator 20 and the oxygen level of the blood after it has passed through the oxygenator 20, an indication of the function of the oxygenator 20 may be provided. This information may be used, e.g., to control the amount of oxygen that is fed into the oxygenator 20 and/or to control the pump 11.

The system 1 may further comprise a bubble trap 70 at or near the blood flow outlet. All blood is emptied into the bubble trap 70 before it leaves to outflow cannula 90. The bubble trap 70 serves to remove any air bubbles from the pumped blood. Bubble trap 70 may include a venting port (not shown) to vent any air entrapped in bubble trap 70. Measures may be provided to increase the dwell time of the pumped blood in the bubble trap 70 to separate smaller air bubbles from the pumped blood. Bubble trap 70 may also include a screen (not shown) to trap any debris from the pumped blood before it is delivered back to the patient P.

A method of using the pump-oxygenator system described herein for (by way of example only) treating heart failure, will be described as follows:
In order to install the system, a patient's vein is percutaneously cannulated with the inflow cannula 80. The inflow cannula 80 may be provided in many different diameters and lengths to suite different patients and different applications. The Seldinger technique may be used to insert the cannula 80 into the femoral vein to drain venous blood into the circuit.

The inflow cannula 80 may then be connected to the circuit of choice. Due to the small volume of the entire circuit, blood may be used to prime the circuit. Usually, saline is used to prime standard cardiopulmonary circuits because of their large volume. Standard circuits volume may range from 100 cc (cubic centimeter; cm³) upward to 1500 cc. The circuit volume of the described system may be well below 100 cc and possible down to 10 cc. The difference in prime volume is a key factor that allows the use of blood to prime the circuit instead of saline. Using patient's blood to prime the system will eliminate the dilution the patient's blood suffers when saline is used. Diluting the patient's blood with saline may reduce the oxygen carrying capacity of the blood such that a higher circulation level is needed to achieve the same tissue oxygenation level.

The outflow cannula 90 may be inserted in the patient in a similar manner as that employed to insert the inflow cannula 80 (i.e., by using the Seldinger technique). The outflow cannula 90 may preferably be primed with the patient's blood. In case the outflow cannula 90 is placed in an artery, a pair of hemostats may be used to control blood flow through the outflow cannula 90.

The primed circuit may then be attached to the primed outflow cannula 90 while keeping the outflow cannula 90 clamped to control flow to or from outflow cannula 90. All cannulas (inflow cannula 80 and/or outflow cannula 90) may be equipped with a quick-connect for quick and ease of attaching the cannula to the circuit. Large amounts of air may be removed at this point through any port such a port of the bubble trap 70.

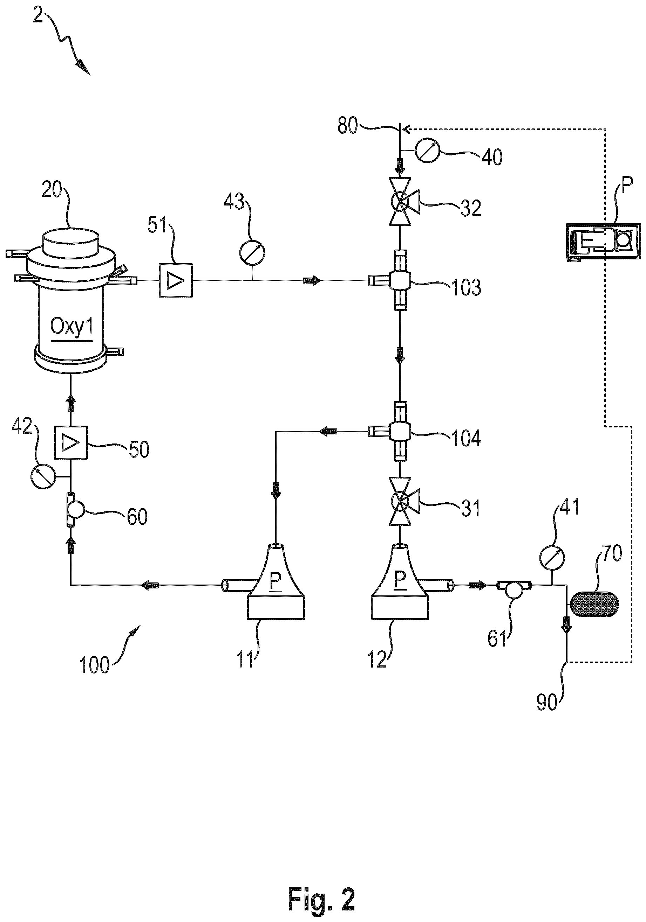

The system 2 according to the embodiment shown in Fig. 2 is substantially similar to that of Fig. 1. Thus, the description of Fig. 1 likewise applies to Fig. 2. However, an additional blood pump 12 is provided between the recirculation loop 100 and the blood flow outlet 90. That means, this extra pump 12 is positioned at or near the outflow cannula 90 to deliver a set volume to the patient. The additional pump 12 may be controlled independently from the blood pump 11 that circulates the blood in the recirculation loop 100. Therefore, the blood volume delivered to the patient P could be instantaneously changed according to the patient requirements. The blood pump 12 may be coordinated with the valve 31, which is located at the input side of the pump 12 in order to provide a delivered flow to the patient P. Specifically, the output pressure must not be too high, i.e. it should not exceed a certain threshold (e.g., 200 mmHg) to avoid harm to the patient P.

A minor difference between the embodiments of Figs. 1 and 2 is the order of the bubble trap 70 and the pressure sensor 41 near the blood flow outlet 90. While the order may be arbitrarily chosen in general, it may be preferred to place the bubble trap 70 as the last feature of the system 1 for the blood to pass before it is delivered back to the patient P.

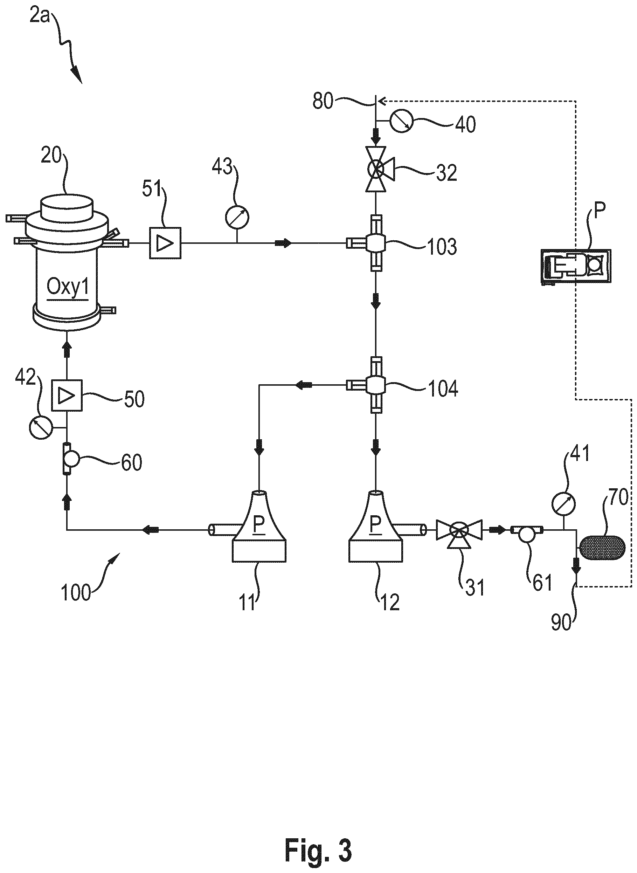

The system 2a according to the embodiment shown in Fig. 3 is substantially similar to that of Fig. 2. Thus, the description of Fig. 2 likewise applies to Fig. 3. However, rather than at the inlet side of the additional pump 12, the valve 31 is placed at the outlet side of the pump 12. This may be advantageous for controlling the output pressure of the pump 12, and may thus be considered a "fine tuning" for the pressure that is actually delivered to the patient P.

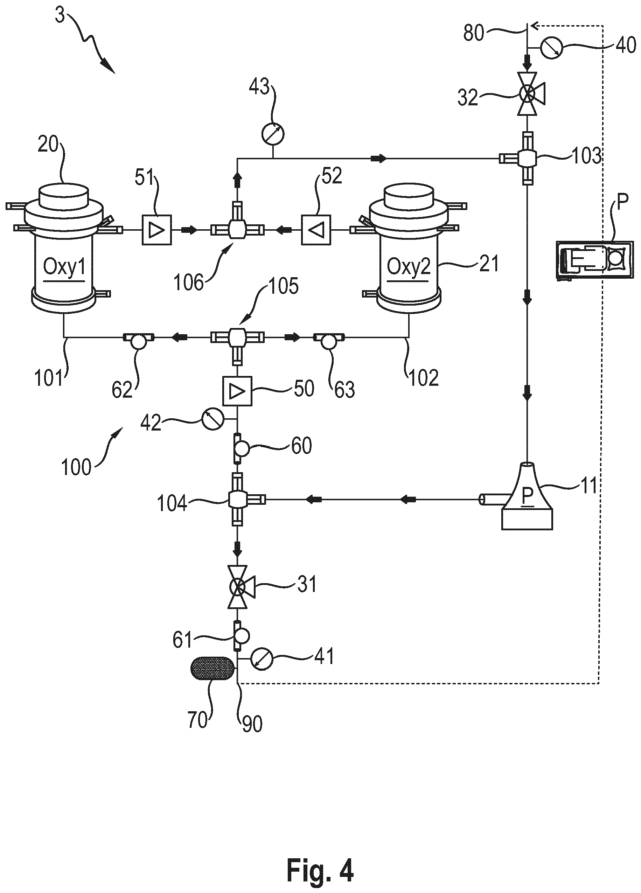

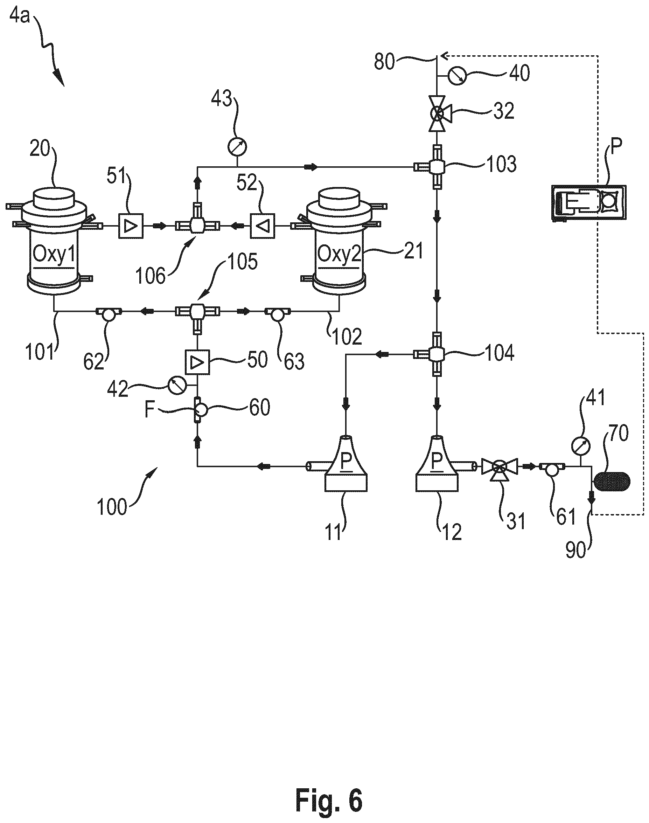

Figs. 4, 5 and 6 show embodiments of blood pump oxygenator systems 3, 4, 4a that are similar to those described above with reference to Figs. 1, 2 and 3, respectively. Thus, the description of Figs. 1 to 3 likewise applies to the respective one of Figs. 4 to 6. However, rather than only one oxygenator 20, two oxygenators 20, 21 are provided. The oxygenators 20, 21 are accommodated in respective parallel branches 101, 102 of the recirculation loop 100. Each branch 101, 102 is equipped with a flow meter 62, 63 as well as oxygen sensor 50, 51, 52 upstream and downstream, respectively, of the respective oxygenator 20, 21. At the inflow side (upstream) of the oxygenators 20, 21 a single oxygen sensor 50 is sufficient before the recirculation loop 100 branches to determine the oxygen level in the blood before passing through one of the oxygenators 20, 21. The oxygen sensors 50, 51, 52 provide information about the oxygen levels as described above.

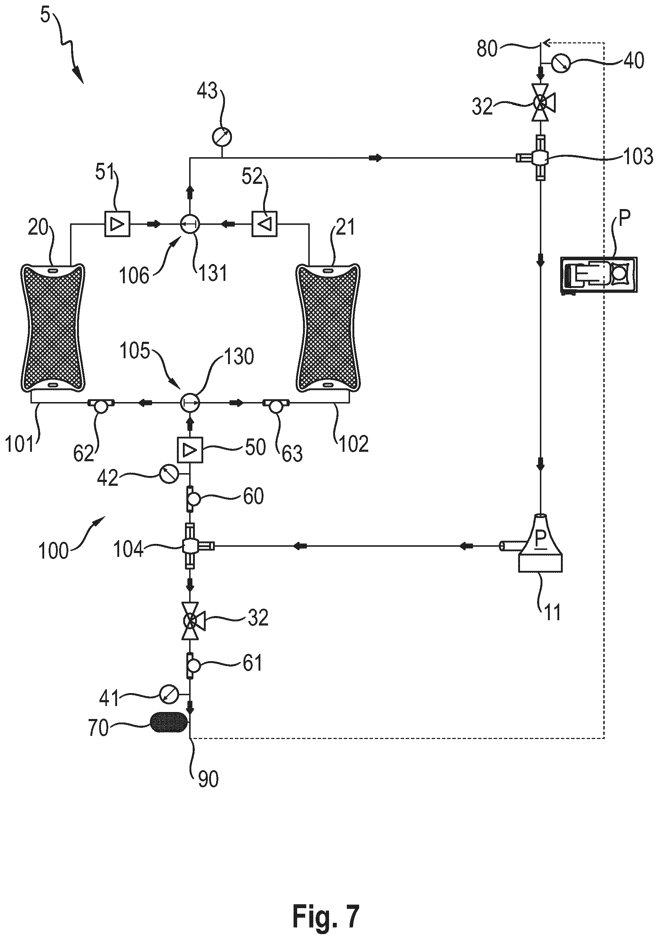

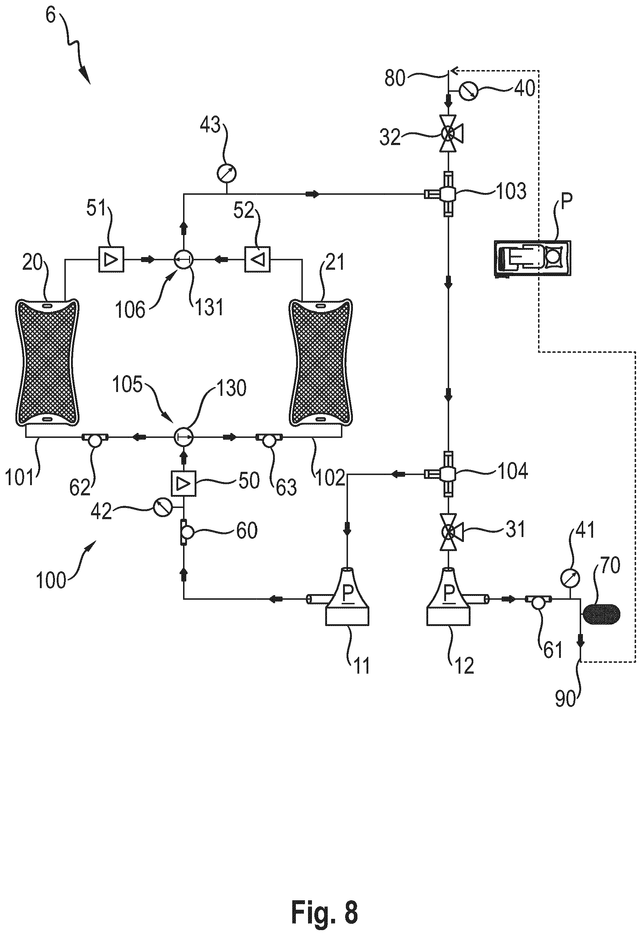

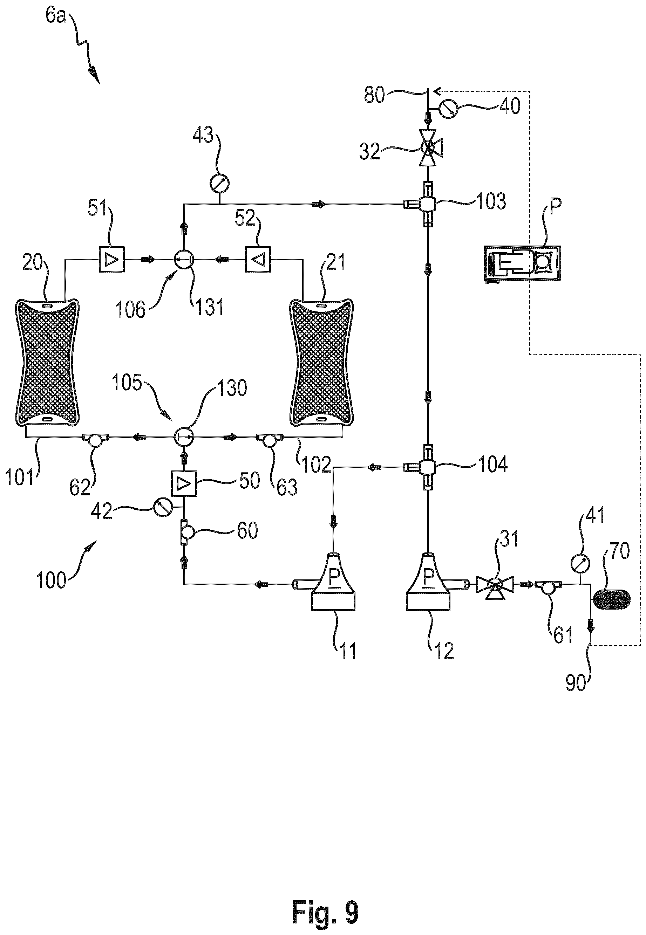

As shown in Figs. 4 to 6, the recirculation loop 100 has a bifurcation 105 where the recirculation loop 100 diverts into the parallel branches 101, 102. The bifurcation 105 may be configured as a simple T- or Y-connectors. Respective valves, such as safety-type valves, may be included in the bifurcation 105 that are open during normal operation and closed, e.g., if one of the branches 101, 102 is disconnected for replacement of the respective oxygenator 20, 21. At the end of the branches 101, 102 the recirculation loop 100 has a junction 106 where the branches 101, 102 reunite leading back into the main section of the recirculation loop 100. While one or both of the bifurcation 105 and the junction 106 may be configured as such simple valves as shown in Figs. 4 to 6, one or both of the bifurcation 105 and the junction 106 may be configured as multi-way control valves 130, 131 as shown, e.g., in Figs. 7 to 9 and described below. One aspect of the pump-oxygenator system 3, 4, 4a having at least two oxygenators 20, 21 is the capability of being able to replace worn oxygenators without stopping the system operation. This eliminates one of the main drawbacks of many known systems, which required the entire system to be stopped in order to replace even one element. This, in turn, required subsequent re-priming of the system after the replacement procedure had taken place. In order to be able to replace an oxygenator 20, 21, a switch may be provided that allows selection of one or more branches 101, 102 of the recirculation loop 100 and, thus, respective oxygenators 20, 21, while disabling an oxygenator that is to be replaced. This "switch out" capability of the system disclosed herein is a significant improvement in that component replacement may be frequently required due to fast degradation of oxygenator elements.

Apart from that, a system having more than one oxygenator is provided with a redundancy to avoid a complete system failure if one oxygenator fails. Furthermore, each of the oxygenators 20, 21 can have a smaller design compared to a system having only one oxygenator. The dwell time of the blood in the oxygenators 20, 21 can be increased.

Now referring to Figs. 7, 8 and 9, embodiments of blood pump oxygenator systems 5, 6, 6a are shown that are similar to those described above with reference to Figs. 4, 5 and 6, respectively. Thus, the description of Figs. 4 to 6 likewise applies to the respective one of Figs. 7 to 9. However, rather than rigid oxygenators, the oxygenators 120, 121 use a flexible shell instead of a hard shell (meaning the oxygenator shell is compliant). Furthermore, in the systems 5, 6, 6a three-way control valves 130, 131 are provided at the bifurcation 105 and the junction 106, respectively.

Generally, a multi-way control valve has multiple ports. For instance, a three-way control valve has three ports. They may also be referred to as selector valves and be configured to act as mixing valves or as diverting valves. The diverting valve 130 is configured to distribute the incoming volume flow to the two branches 101, 102 at the bifurcation 105. The mixing valve 131 mixes the blood coming from the branches 101, 102 at the junction 106. The two selector valves 130, 131 may be configured to select one (flexible) oxygenator 120, 121 or the other at a time (and, thus, may be considered acting as switches). One possibility would be to select one of the branches 101, 102, and thus one of oxygenators 20 ,21 and to block the other one. In particular, in Figs. 7 to 9, the valves 130, 131 are in a switching position in which blood flows through the branch 102, while the branch 101 is bypassed. Another possibility would be to fill one oxygenator while its output is closed, and at the same time emptying the other into the patient. In other words, one (soft shell) oxygenator is inflating with blood while the other is emptying the oxygenated blood into the patient, thus the pump 11 is inflating one oxygenator while the other oxygenator is deflating the oxygenated blood into the patient. It will be appreciated that such multi-way control valves may be provided in each of the embodiments, in which the recirculation loop 100 includes at least two oxygenators 20, 21 arranged in parallel branches 101, 102.

This procedure can increase the dwell time of blood inside each oxygenator 120, 121 (it can be from 3 to 200 seconds before any blood clotting). Therefore, the oxygen volume could be decreased and still attain a similar oxygenation level due to an increase in dwell time. Using this method, the whole circuit can act as a pulsatile system that changes the blood flow inside the oxygenators with each cycle of filling and emptying. In addition, the increase in dwell time will allow for better bubble detection and removal. Also, due to the flexible design of the oxygenators 120, 121, and thus movement of the oxygenators 120 ,121, the blood may tend to take different paths through the oxygenators 120, 121. This may increase the efficiency of the oxygenators 120, 121 because more or even all fibers of the oxygenators 120, 121 are used regularly compared to a stiff and rigid oxygenator design.

In some embodiments, as will be described also in more detail below, there is at least one flexible oxygenator placed inside another flexible bag. The features described above, specifically with reference to Figs. 7 to 9 may likewise apply to the following embodiments. It is to be noted that the flexible bag could be pressurized (i.e., squeezed) to force empty the flexible oxygenator. In other words, the flexible oxygenator may be emptied by air pressure outside it rather than a rotary pump use. Embodiments of flexible, and thus wearable (portable), oxygenator systems are described below. This provides a great improvement for the patient's mobility and reduction of pain. Furthermore, since the blood in the wearable device may be close to the patient's body (e.g., on the chest and/or back of the patient), the blood may be maintained at a desired temperature without the need of an additional heat exchanger.

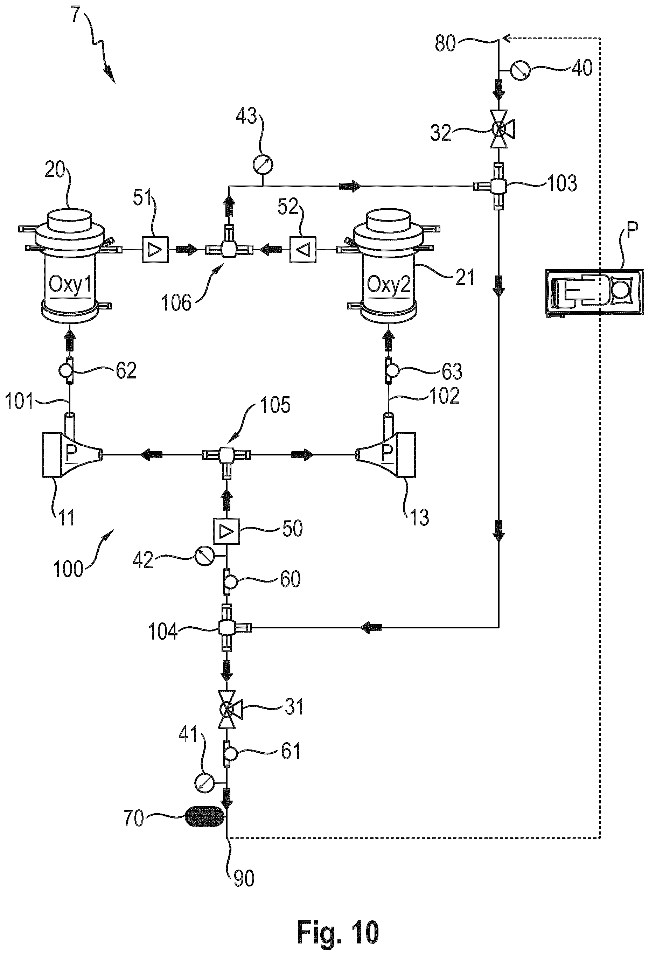

Fig. 10 shows an embodiment of a blood pump oxygenator system 7 that is similar to that described above with reference to Fig. 4. Thus, the description of Fig. 4 likewise applies to Fig. 10. However, rather than having a single pump 11 in the main section of the recirculation loop 100 (i.e., upstream of the bifurcation 105), a pump is arranged in each of the branches 101, 102. More specifically, pump 11 is located in branch 101 between the bifurcation 105 and oxygenator 20, while pump 13 is located in branch 102 between the bifurcation 105 and oxygenator 21. Thus, each of the pumps 11, 13 is configured to feed one of the oxygenators 20, 21. The system 7 improves safety for the patient as it provides more redundancy and increased safety when changing a pump. Each branch 101, 102 provides full functionality and can continue working while components in the other branch can be replaced.

Each branch 101, 102 further comprises a flow meter 62, 63 before each oxygenator 20, 21 and an oxygen sensor 51, 53 after each oxygenator 20, 21 as described above. This configuration allows to provide a blood pump-oxygenator unit in each branch 101, 102, each unit comprising a pump, flow meter, oxygenator and oxygen sensor. Such unit can be replaced as a whole, which may facilitate handling of the system, e.g., allows for faster switching, and may increase overall lifetime. In particular, not only the oxygenators 20, 21 may wear over time but also the blood pumps 11, 13, which is why it is advantageous to make also the blood pumps 11, 13 disposable. Respective valves, such as safety-type valves, may be included in the bifurcation 105 that are closed, e.g., if one of the pumps 11, 13 stops or is turned off to disconnect a respective one of the branches 101, 102 for maintenance or replacement.

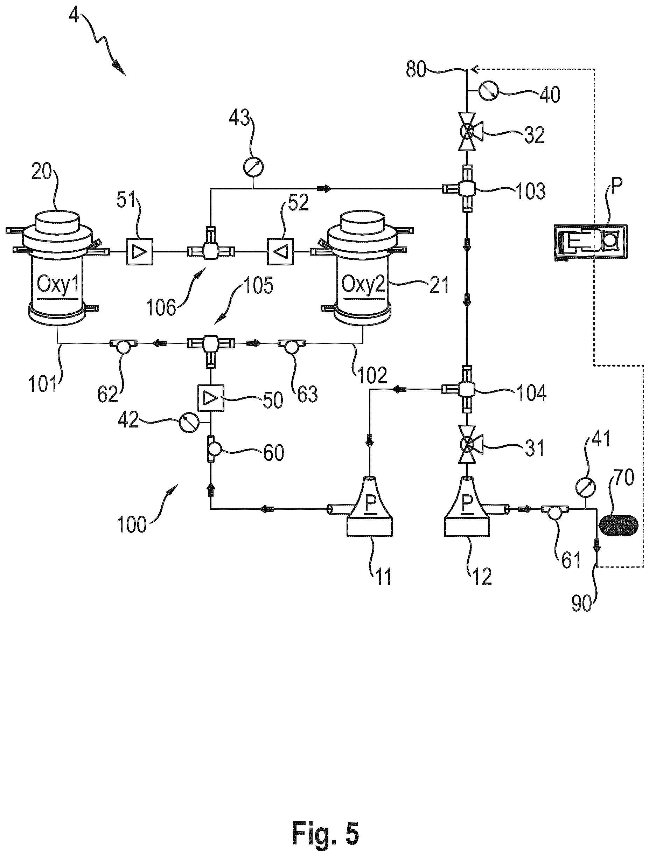

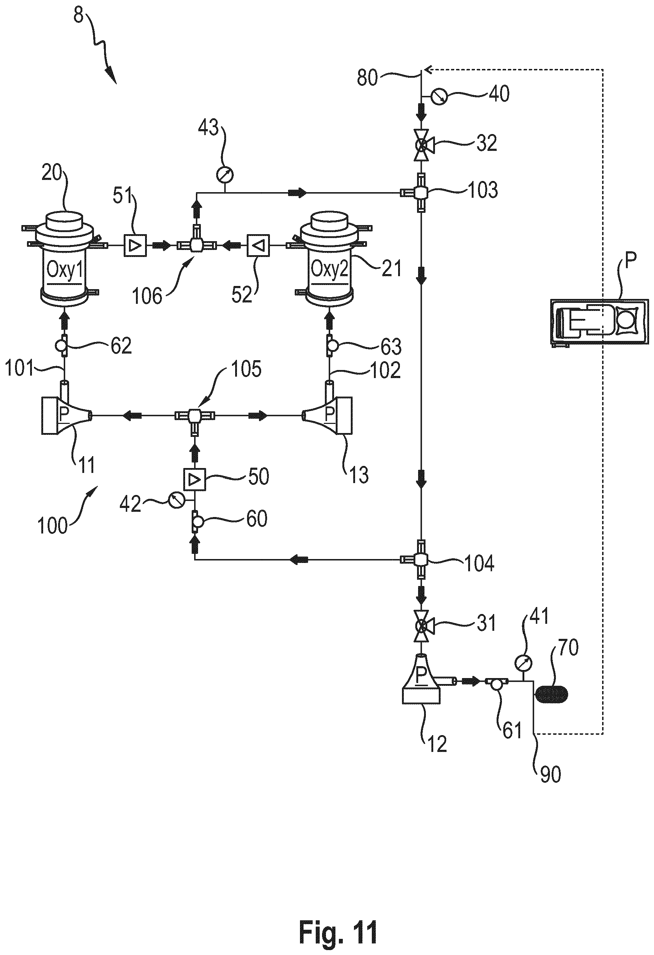

Fig. 11 shows an embodiment of a blood pump oxygenator system 8 that is similar to that described above with reference to Fig. 5. Thus, the description of Fig. 5 likewise applies to Fig. 11. Also, the system 8 is similar to the system 7 described above with reference to Fig. 10. Thus, the description of Fig. 10 likewise applies to Fig. 11, particularly with regards to the arrangement of the pumps 11 and 13. In other words, the system 8 of Fig. 11 combines the arrangement of pumps 11, 13 in each of the branches 101, 102 of the recirculation loop 100 with a further pump 12 at the blood flow outlet 90 and, thus, comprises three pumps 11, 12, 13 in total.

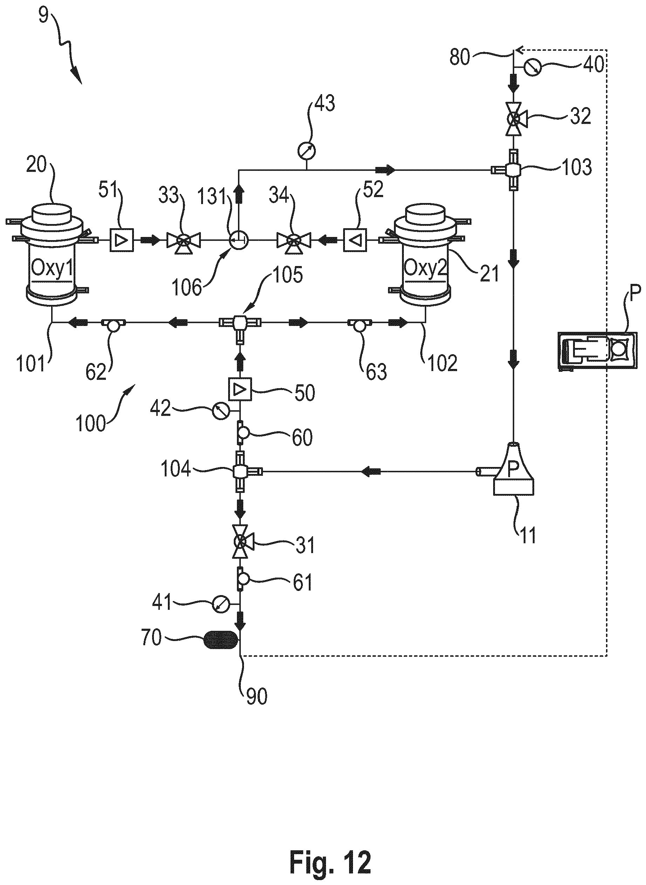

Fig. 12 shows an embodiment of a blood pump oxygenator system 9 that is similar to that described above with reference to Fig. 4. Thus, the description of Fig. 4 likewise applies to Fig. 12. However, the system 9 includes an improved valve assembly at the junction 106. More specifically, the valve assembly includes a switch 131 (e.g., mixing valve as described above) along with automatically controlled valves 33, 34 at the respective ends of the branches 101, 102, i.e., at the inputs of the switch 131. This configuration may provide the advantages of a pulsatile system described above with respect to Figs. 7, 8 and 9. For instance, the valves 33, 34 along with the switch 131 can be controlled in a pulsatile manner. While one of the valves 33, 34 is closed, which causes the respective oxygenator 20, 21 to fill up, the other one of the valves 33, 34 can be opened to empty the respective oxygenator 20, 21. This can be done by a time-dependent control of the valves, e.g., switching between the branches every 2 seconds.

Alternatively, the valves can be controlled in accordance with the oxygen level measured by the oxygen sensors 51, 52, respectively. For instance, the valves 33, 34 can be kept closed until a desired oxygen level in the respective oxygenator 20, 21 is reached, and then opened to empty the respective oxygenator 20, 21. This may allow to maximize the dwell time of blood in the oxygenators 20, 21 (generally, it will be understood that a maximum dwell time is limited by the clotting time of the blood).

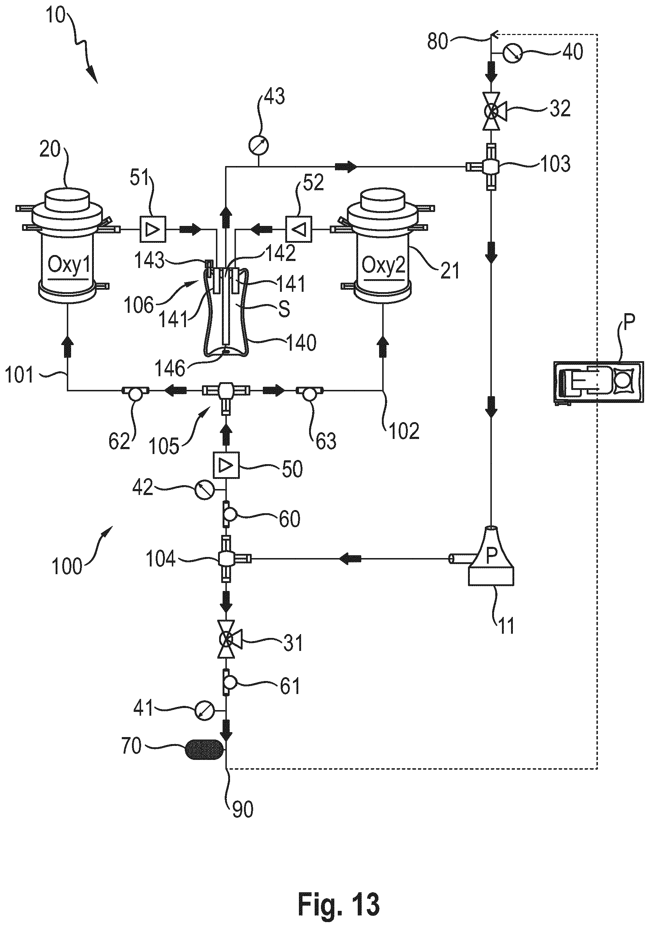

Fig. 13 shows an embodiment of a blood pump oxygenator system 10 that is similar to that described above with reference to Fig. 4. Thus, the description of Fig. 4 likewise applies to Fig. 13. However, the system 10 includes a specific arrangement at the junction 106, which may be referred to as reservoir (or "blood collecting device") 140. It will be appreciated that any embodiment may be provided with such reservoir 140, including those that have only one oxygenator (see, e.g., Figs. 1 to 3). Blood enters the device 140 from all branches 101, 102 via inlet ports 141 and exits the device 140 via an outlet port 142. Gas bubbles may be exhausted at a vent 143.

As gas bubbles will move upwards, the outlet port 142 has an exit point 146 which is at a distance from the vent 143 towards the inside of the device 140. This may be achieved, e.g., by means of a tubing which extends the outlet port 142 towards the inside of the device towards a lower portion of the device 140 with an end of the tubing forming the exit point 146 for the outlet port 142. It will be appreciated that the device 140 must be in an upright (i.e., vertical, or at least substantially vertical) position in which the vent 143 is at the top of the device 140. At least, the vent 140 must be above the exit point 146 of the outlet port 142. Once the device 140 is completely filled, the occurring inner pressure will cause the blood to exit the device 140 through the exit point 146 and the outlet port 142.

The reservoir 140 may be designed with a flexible outer shell, such as a flexible bag or sack. This allows emptying the reservoir 140 by squeezing it, which reduces blood damages. The squeezing may be done either manually or by means of a mechanical squeezing device (not shown), which may, e.g., synchronized with the patient's heartbeat. The reservoir 140 may be a blood unit, wherein a heater may be provided on a back cover or lid.

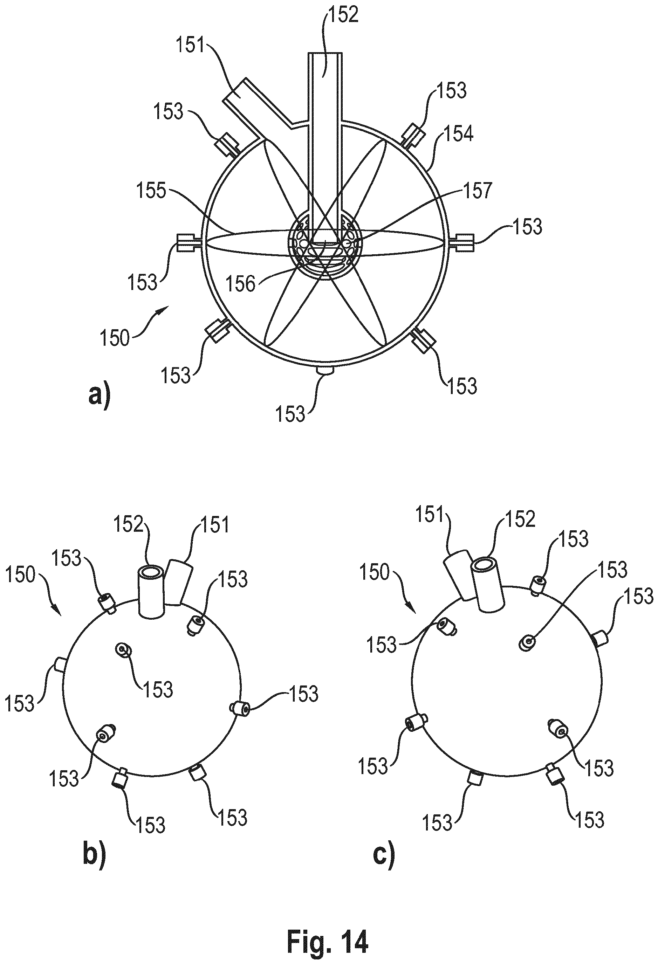

Fig. 14 shows an embodiment of a reservoir 150. It will be appreciated that it could replace the reservoir 140 of the system 10 shown in Fig. 13. The device 150 is shown in a cross-sectional view (a) as well as in views from different angles (b, c).

While the reservoir 140 described above with reference to Fig. 13 is generally suitable for a wearable device due to its flexible bag-like configuration, it must be in an upright position during operation to keep the vent oriented upwards as described above with the exit point 146 of the outlet port 143 being below the vent 143. However, particularly with regards to a wearable device, the position and orientation may change as the patient moves, e.g., lays down. Thus, it might not always be ensured that the vent 143 of the device 140 faces upwards. The reservoir 150 shown in Fig. 14, thus, has a design which ensures proper function independent from its orientation. Apart from that, the function of the device 150 is similar to that of the device 140.

The reservoir 150 has one inlet port 151. More than one inlet port may be provided depending on the number of oxygenators of the blood pump-oxygenator system, in which the device 150 is intended to be used. An outlet port 152 is provided with an exit point 156 that is substantially in the center of the device 150. A plurality of vents 153 is provided distributed along the outer circumference of the device 150. The number and position of vents 153 may vary as long as at least one of the vents 153 can be positioned substantially at the top of the device 150 in any orientation of the device 150. This configuration ensures that in any orientation of the device 150 at least one of the vents 153 is above the exit point 156 of the outlet port such that gas bubbles can escape. In order to retain any debris, such as blood clots, and to avoid such debris to be conveyed through the system, a filter 155, such as a mesh- or sponge-like structure may be provided covering the exit point 156 of the outlet port 152.

The reservoir 150 has a ball shape, particularly a spherical shape. It will be appreciated, however, that any shape may be envisioned as long as the described arrangement of a plurality of vents, inlet port(s) and outlet port is provided. In order to allow the device 150 to be squeezed as described above with respect o the device 140 it may have a flexible outer shell 154. A biasing arrangement, such as metallic rings 155 may be provided to allow the device 150 to return to its original shape. It will be appreciated, however, that the device 150 may alternatively have a hard outer shell. In any case, blood will be pushed out of the outlet port 142 once the device 150 is completely filled.

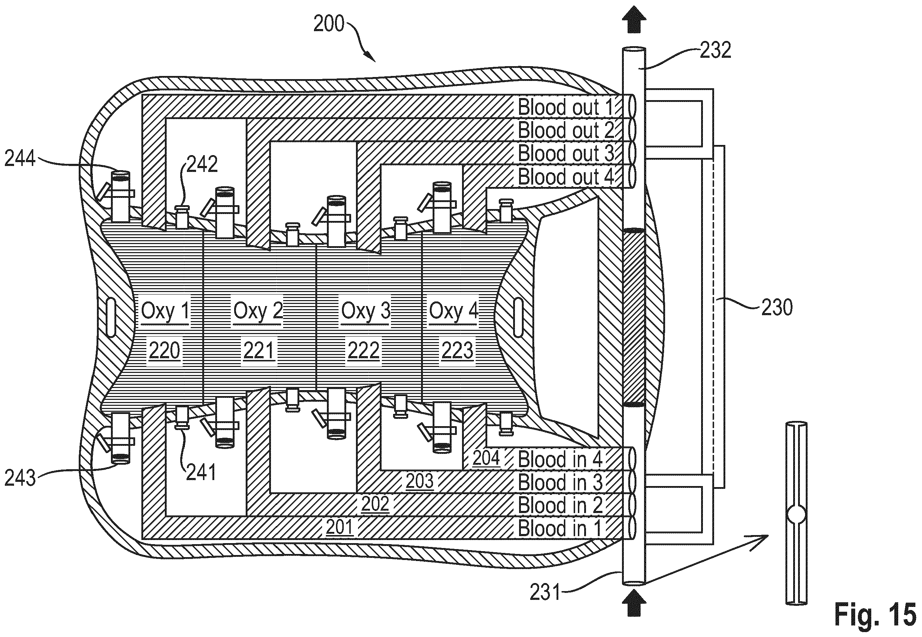

The embodiment shown in Fig. 15 includes a flexible bag 200 with four separate chambers, each of which accommodates an oxygenator 220, 221, 222, 223. Thus, the device of Fig. 15 has four oxygenators, which are arranged in four parallel paths 201, 202, 203, 204. A mechanical switch 230 is provided to allow for selecting one or more of the four oxygenators 220, 221, 222, 223, while the respective other oxygenator(s) are blocked. Thus, once an oxygenator is to be replaced, it can simply be taken out of service, while at least one of the other oxygenators continues to run. As shown in the detail of Fig. 15, the switch 230 may basically comprise a tubular member 231, 232 with at least one cutout for putting the inlet in connection with one of the oxygenators. Both tubular members 231, 232 are moved in a coupled fashion to automatically select the correct outflow path.

Each of the oxygenators 220, 221, 222, 223 further has a priming inlet 241 and priming exhaust port 242 which allows priming the respective oxygenator before its actual use. For instance, saline may be pushed into the oxygenator chamber by means of a syringe to purge out all air. Once all air has been removed by priming the oxygenator, the priming inlet 241 and priming exhaust ports 242 are closed, and blood is let into the oxygenator chamber. Oxygen is fed into the oxygenator by a respective gas inlet 243, while carbon dioxide (as well as unused oxygen) leaves the oxygenator via a respective gas outlet 244.

It will be appreciated that with regards to the systems described above, the device of Fig. 15 may be considered to form an "oxygenator part" of the recirculation loop. It will be appreciated that the device is configured to be connected to respective inflow and outflow cannulas, pump(s), valve(s), sensor(s) are described above.

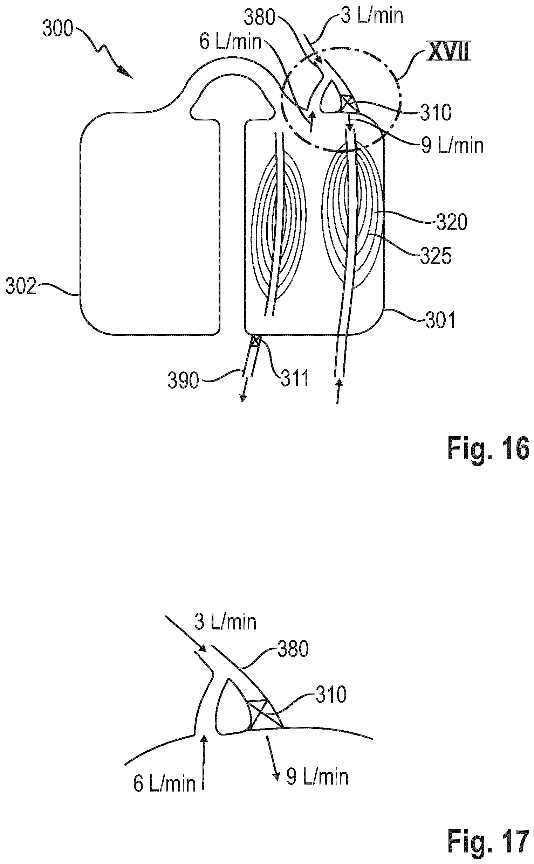

In one embodiment as shown in Fig. 16, a wearable soft-shell pump-oxygenator system is provided in the form of a wearable vest 300, comprising a blood oxygenator 320 placed inside the vest, composed of hollow fibers 325, between a first pump 310 and a second pump 311, wherein an inlet of the first pump 310 is connected to the patient's vein and an outlet of the second pump 311 is connected to patient's artery. The circuit provides continuous blood communication between components. The pump operation could be cyclically operated in timed relation to patient's heartbeat or one of the physiological parameters, and a control circuit varies the velocity of the pump function in response to changes in the said physiological parameter. The device itself, controller, drive line and power supply can be worn by the patient so that the patient can be ambulatory. Blood enters through an inflow cannula 380, gets oxygenated through the hollow fiber area that is spread in almost complete area of the left side 301 of the vest 300, which has a capacity of around half or 0.6 liters. Around 3 L/min of oxygenated blood is delivered to the patient through a pump 311 placed in the outflow cannula 390 or at the bottom of the left side 301 of the vest 300. The pump 310 that delivers blood to the oxygenator 320 will deliver around 9 L/min, 3 L coming from patient and 6 L from the device inside the vest 300 in a recirculation mode.

Fig. 17 is a zoom view of the first pump circuit of the embodiment of Fig. 16. According to this embodiment, an amount of 3 L/min of blood may come from the patient, and in turn 3 L/min may be delivered back to the patient. Apart from that, 6 L/min of blood are recirculating through the hollow fibers 325 of the oxygenator 320 inside the left side 301 of the vest 300, thus, in total 9 L/min.

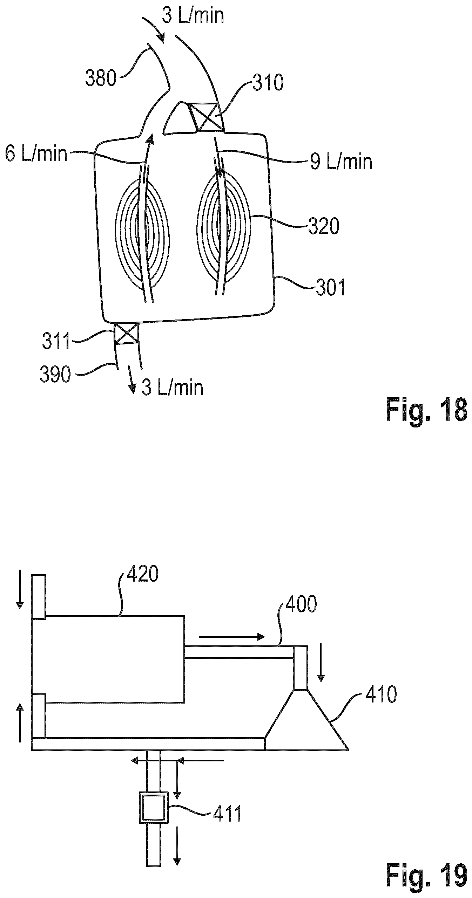

Fig. 18 is a detailed view of the left side 301 of the vest with inflow and outflow of blood correspondingly. Also the first and second pumps 310, 311 are schematically illustrated, one of which draws the blood into the oxygenator 320, and the other one returns the blood from the oxygenator 320 to the patient.

Now referring to Fig. 19, recirculating system 1 is provided comprising a recirculating pump 410, an infusion pump 411, and a recirculation loop 400 with an oxygenator 420. The extracorporeal system may boast a ready to use circuit comprising a recirculating pump 410, an infusion pump 411, and recirculation loop 400 to allow improved performance and ease of use of existing oxygenators in extracorporeal oxygenation. The recirculating system is intended to be used in conjunction with any extracorporeal oxygenation system to improve the performance of increase oxygenation while using a smaller oxygenator.

The recirculating pump 410 may be provided (by way of example only) as a rotary or a displacement pump and, more specifically, a rotary pump of the centrifugal type. Centrifugal pumps are a known art in the blood pumping area and are preferred due to the high flow rate and the low trauma to the blood. The recirculating pump 410 may be directly driven by an electric motor or driven by a flexible cable that links the pump to the electric motor (as described, for example, in US 4,944,722). The coupling between the pump and the driving mechanics may be magnetically or directly coupled. The pump 410 is shown of the centrifugal flow type with a rotor situated inside a pump housing, with a drive cable inside a sheath coupled to the rotor (not shown). The rotation of the cable, by an electric motor (not shown), causes the rotation of the rotor and the pumping of fluid from the distal end to the proximal end of the housing. In this fashion, fluid is transported from a pump inflow towards a pump outflow and passes through the oxygenator continually at the maximum flow rate allowed by oxygenator. The pump 410 may provide a pump rate of 8 to 12 L/min.

The infusion pump 411 may be provided (by way of example only) as a rotary or a displacement pump and, more specifically, a rotary pump of the axial type. Axial pumps are a known art in the blood pumping area and are preferred due to the moderate flow rate and the low trauma to the blood. The infusion pump 411 may be directly driven by an electric motor or driven by a flexible cable that links the pump to the electric motor (as described, for example, in US 4,944,722). The coupling between the pump and the driving mechanics may be magnetically or directly coupled. The infusion pump 411 is shown of the axial flow type with a rotor situated inside a pump housing, with a drive cable inside a sheath coupled to the rotor (not shown). The rotation of the cable, by an electric motor (not shown), causes the rotation of the rotor and the pumping of fluid from the distal end to the proximal end of the housing. In this fashion, fluid is transported from a pump inflow towards a pump outflow and passes to the circuit delivering blood to the patient (not shown). The pump 411 may provide a pump rate of 2 to 4 L/min.

The recirculating system could be used in conjunction with any cardiopulmonary bypass system (CPB) or any extra corporeal membrane oxygenation (ECMO) to increase the oxygenation of blood with a smaller oxygenator size. Using a smaller oxygenator size will decrease the priming volume of the circuit, therefore, it will decrease blood dilution and increase blood capability to carry more oxygen to the patient. In addition, the recirculating system will allow the use of ambient air instead of pure oxygen and will attain a similar level of oxygenation. Eliminating the use gas with high oxygen concentration will eliminate the risk associated with flammability of concentrated oxygen and, therefore, will allow the use of the recirculating system in environment outside hospital setting, such as ambulance and environment where highly concentrated oxygen is not available.

In the following, some basic components of the oxygenator are described in more detail with reference to Figs. 20 to 23. The oxygenator 20 may have hollow fibers 25 used in known oxygenators. They may be hair-like fibers. The fibers may be arranged in "sets" or "bundles", wherein fibers of one set may have a common inlet and outlet respectively. The wearable device, thus, may have an extraluminal flow regime, where the blood flows outside the gasfilled hollow fibers 25. In other words, the blood taken from the patient fills the volume of the vest, either partially or substantially completely to flow around the hollow fibers for oxygenation and removal of carbon dioxide, which is generally known to the skilled person. However, the wearable system for the first time provides a flexibility of the overall components such that a wearable device is provided to allow mobility of the patient.

Fig. 20 is a detailed view of two sets of hollow fibers 25 shown in a cross-sectional view, where oxygen passes in the middle of hollow fibers 25 around them. Fig. 21 is a detailed view of one set of hollow fibers 25 showing where the flow of oxygen occurs in a path 28, and the carbon dioxide coming out in a side path 29. Fig. 22 shows a side path 29 of CO₂ out. Fig. 23 shows another structure where CO₂ side 29 path is bigger than in Fig. 22.

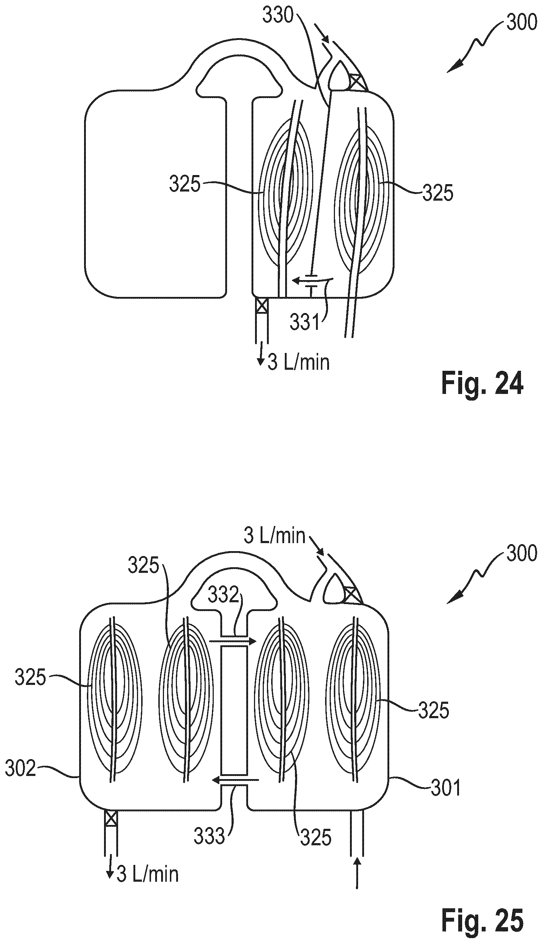

Referring now to Figs. 24 to 30, various embodiments of wearable oxygenator system in the form of a vest 300 are described. It will be appreciated that features described above with reference to Figs. 20 to 30 may likewise be applicable.

Fig. 24 shows an embodiment where there is a partition 330 between the two sets of hollow fibers 325, blood will circulate from one side to the other through a small opening 331. This way is designed to ensure that blood will also flow in the second set of hollow fibers not only the first one.

Fig. 25 shows another embodiment where there are multiple sets of hollow fibers 325, preferably four, two in each side 301, 302 of the vest 300. The skilled person will understand that any suitable numbers of sets of hollow fibers 325 may be placed inside the vest 300 depending on the size and the specific needs. The number of sets in the left and rights sides 301, 302 may be the same or different. Openings, cross-links or other suitable connection elements between the two sides 301 ,302 (i.e., the left and the right sides, or possibly front and back sides) of the vest 300, e.g., two openings 332, 333, preferably one in the upper portion and one in the lower portion, may be provided to allow for recirculation of blood substantially all over the area of the vest 300. This allows to enhance oxygenation of the blood before it is returned to the patient blood circulation, thereby providing an efficient wearable oxygenator.

One generally important aspect of the present disclosure is to ensure that blood is passing through the largest surface of hollow fibers 325 in order to ensure higher oxygenation level and to ensure a longer life span of the hollow fibers. Or in other words, the design of the vest 300, including respective cross-links, connection elements, openings or the like is such that as much as possible of the volume of the vest can be utilized to increase efficiency. Also providing an amount of recirculation of the blood inside the vest 300 improves efficiency as the blood may pass along the fibers more than one time. These aspects also contribute to making possible the wearable design.

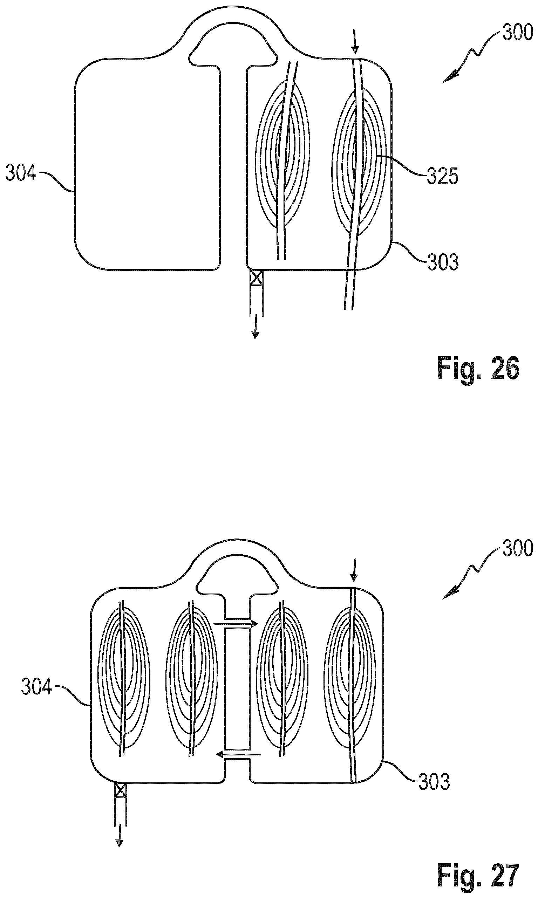

Fig. 26 shows another embodiment of the vest 300 having hollow fibers 325 in the back side 303 of it as a backup in case the fibers in the front of the vest 300 need to be changed. It will be appreciated that the backside may not only be provided as backup but may take the role of the front side as in the other embodiments. Embodiments may be envisioned in which both, the front and back side are utilized to increase efficiency.

While the right and left sides 303, 304 in the embodiment of Fig. 26 are not interconnected, Fig. 27 shows a similar embodiment to that of Fig. 26 but the fibers are in right and left sides 303, 304 of the back side of the vest 300 with circulation of blood between the left and right.

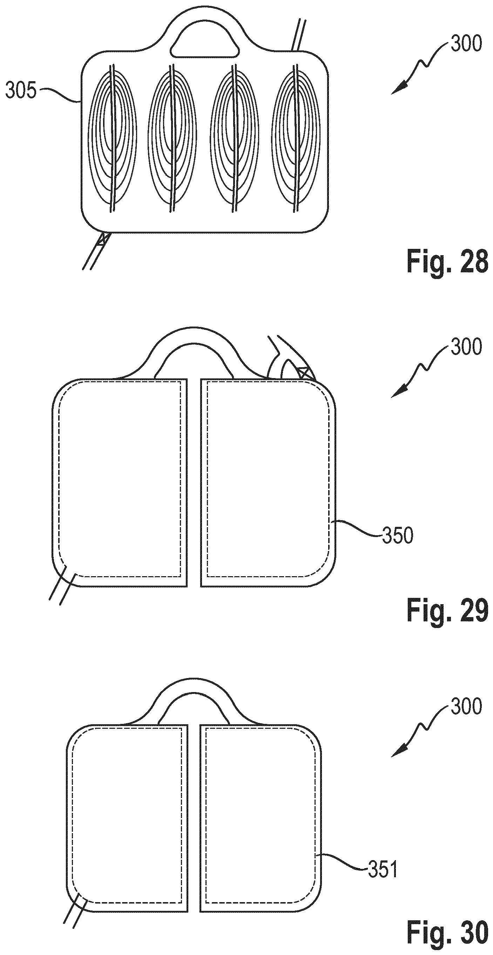

Rather than having the left and right sides 303, 304 separated or connected via connection elements or openings, Fig. 28 shows another embodiment where the back side 305 of the vest 300 is one piece that has hollow fibers all over. The skilled person understands that this may be applied also to the front side. Generally, it is advantageous to utilize substantially or at least a major portion the entire volume of the vest for recirculation of the blood to increase efficiency.

Fig. 29 shows an embodiment in which a hard cover 350 is added to the device over the front vest 300 in order to protect the components inside the vest. Nevertheless, the device is still designed to be wearable but may be more robust compared to a device with a completely soft outer shell.

Fig. 30 shows an embodiment in which a hard cover 351 is added also over the back side of the vest 300 in case it contains components. It protects the device in case the patient is sleeping on his back for example.

It will be appreciated that the device may accomplish partial or total gas exchange (O₂ and CO₂) without cardiac support allowing the lungs to rest in case needed in order to heal. This may or may not be driven by a pump.

It will be appreciated that in accordance with any embodiment described herein, an electronic controller may be required that controls the features of the system (such as pump speed, oxygen gas volume delivery, valve opening and closing) as well as the diagnostic sensors (pressure sensors, oxygen sensors, flow meters) that indicate proper performance of the system. More specifically, the components of the system may be controlled in such a way that the system may be automatically controlled by respective feedback loops, e.g., the pump(s) and valve(s) may be controlled using sensor data from the diagnostic sensors. The specifics of such controller are common to those skilled in the art.

While above at least one exemplary embodiment of the present invention has been described, it has to be noted that a great number of variations thereto exists. Furthermore, it is appreciated that the described exemplary embodiments only illustrate non-limiting examples of how the present invention can be implemented and that it is not intended to limit the scope, the application or the configuration of the herein-described apparatuses and methods. Rather, the preceding description will provide the person skilled in the art with constructions for implementing at least one exemplary embodiment of the invention, wherein it has to be understood that various changes of functionality and the arrangement of the elements of the exemplary embodiment can be made, without deviating from the subject-matter defined by the appended claims and their legal equivalents.

## Claims

1. A blood pump-oxygenator system (1; ...; 10) for increasing perfusion and oxygen level in a patient (P), comprising a first blood pump (11), at least one oxygenator (20, 21), a blood flow inlet (80), and a blood flow outlet (90), which are connected so as to form a circuit to be operable as a cardiopulmonary bypass system adapted for extracorporeal processing of the patient's blood, wherein the first blood pump (11) is configured to convey blood through the circuit from the patient (P) into the blood flow inlet (80), through the oxygenator (20, 21) and out of the blood flow outlet (90) back into the patient (P), wherein the system (1; ..., 10) further comprises:
a recirculation loop (100) arranged between the blood flow inlet (80) and the blood flow outlet (90), wherein the recirculation loop (100) accommodates the first blood pump (11) and the oxygenator (20, 21), wherein the first blood pump (11) is configured to circulate at least part of the blood in the circuit through the recirculation loop (100) such that the blood passes through the oxygenator (20) multiple times, and
at least a first valve (31) positioned between the recirculation loop (100) and the blood flow outlet (90) and configured to control an amount of blood flowing through the recirculation loop (100).

2. The blood pump-oxygenator system of claim 1, further comprising a second blood pump (12) positioned between the recirculation loop (100) and the blood flow outlet (90) and configured to deliver a set volume to the patient (P), wherein the second blood pump (12) is preferably configured to be controlled independently from the first blood pump (11) that circulates the blood in the recirculation loop (100).

3. The blood pump-oxygenator system of any one of the preceding claims, wherein the first blood pump (11) is configured to be set to deliver a flow rate in excess of what is desired to deliver to the patient (P), wherein the excess amount of blood is recirculated through the recirculation loop (100) and the oxygenator (20).

4. The blood pump-oxygenator system of any one of the preceding claims, comprising at least two oxygenators (20, 21), wherein the recirculation loop (100) comprises a bifurcation (105) that diverts the blood flow in the recirculation loop (100) into at least two branches (101, 102) arranged in parallel with each other and a junction (105) where the at least two branches (101, 102) are brought together, wherein one of the at least two oxygenators (20, 21) is arranged in each of the at least two branches (101, 102).

5. The blood pump-oxygenator system of claim 4, wherein at least one of the bifurcation (105) and the junction (106) is configured as a multi-way control valve (130, 131) that is operable so as to select at least one of the at least two branches (101, 102) for the blood to flow through.

6. The blood pump-oxygenator system of claim 4 or 5, wherein the first blood pump (11) is arranged in the recirculation loop (100) upstream of the bifurcation (105), or wherein the system comprises at least two of the first blood pump (11, 12), wherein one of the at least two first blood pumps (11, 12) is arranged downstream of the bifurcation (105) in each of the at least two branches (101, 102), preferably in each of the at least two branches (101, 102) between the bifurcation (105) and the respective one of the at least two oxygenators (20, 21).

7. The blood pump-oxygenator system of any one of claims 4 to 6, wherein a reservoir (140; 150) is provided at the junction (106)reservoir, the reservoir (140; 150) comprising at least two inlet ports (141; 151), wherein each of the branches (101, 102) ends in the reservoir (140; 150) at a respective inlet port (141; 151), an outlet port (142; 152) for the blood to exit the reservoir (140; 150), and at least one vent (143; 153) configured for exhausting gas bubbles in the blood in the reservoir (140; 150), wherein the outlet port is extended towards an inside of the reservoir (140; 150).

8. The blood pump-oxygenator system of any one of the preceding claims, further comprising a first oxygen sensor (50) and a second oxygen sensor (51) both arranged in the recirculation loop (100) and configured to measure an oxygen level in the blood, wherein the first oxygen sensor (50) is positioned upstream of the at least one oxygenator (20, 21) and the second oxygen sensor (51) is positioned downstream of the oxygenator (20, 21) so as to obtain an oxygenation rate of the oxygenator (20, 21) by a comparison of an oxygen level measured by the first oxygen sensor (50) with an oxygen level measured by the second oxygen sensor (51).

9. The blood pump-oxygenator system of claim 8, wherein the first blood pump (11) is configured to be controlled such that a pump rate of the first blood pump (11) is set depending on the obtained oxygenation rate, wherein preferably a pump rate of the first blood pump (11) is increased when the oxygenation rate decreases.

10. The blood pump-oxygenator system of claim 8 or 9, wherein the first valve (31) is configured to be controlled such that an amount of blood flowing through the recirculation loop (100) is set depending on the obtained oxygenation rate, wherein preferably a flow rate of the first valve (31) is decreased when the oxygenation rate decreases.

11. The blood pump-oxygenator system of any one of the preceding claims, further comprising a second valve (32) positioned between the blood flow inlet (80) and the recirculation loop (100) and configured to control an amount of blood flowing from the patient (P) into the circuit.

12. The blood pump-oxygenator system of any one of the preceding claims, further comprising at least one of a first pressure sensor (40) and a second pressure sensor (41), wherein the first pressure sensor (40) is positioned at the blood flow inlet (80) and configured to measure an input pressure of the circuit and the second pressure sensor (41) is positioned at the blood flow outlet (90) and configured to measure an output pressure of the circuit, wherein the first blood pump (11) is configured to be controlled such that a pump rate of the first blood pump (11) is set depending on the measured input pressure, wherein preferably a pump rate of the first blood pump (11) is increased when the input pressure drops to a predefined threshold.

13. The blood pump-oxygenator system of any one of the preceding claims, further comprising a third pressure sensor (42) and a fourth pressure sensor (43) both arranged in the recirculation loop (100), wherein the third pressure sensor (42) is positioned upstream of the at least one oxygenator (20, 21) and the fourth pressure sensor (43) is positioned downstream of the oxygenator (20, 21) so as to detect a pressure drop caused by the oxygenator (20, 21) by a comparison of a pressure measured by the third pressure sensor (42) with a pressure measured by the fourth pressure sensor (43).

14. The blood pump-oxygenator system of any one of the preceding claims, further comprising at least one flow meter to measure an amount of blood flow, the at least one flow meter comprising at least one of a first flow meter (60) and a second flow meter (61), the first flow meter (60) arranged in the recirculation loop (100), preferably between the first blood pump (11) and the at least one oxygenator (20, 21), and configured to measure a flow rate of blood in the recirculation loop (100), and the second flow meter (61) arranged between the recirculation loop (100) and the blood flow outlet (90) and configured to measure a flow rate of blood that is delivered back to the patient (P).

15. The blood pump-oxygenator system of any one of the preceding claims, wherein the system is provided as a wearable soft shell extracorporeal pump-oxygenator system.
